# EUROPEAN PATENT APPLICATION

(11) **EP 4 506 346 A1**
(43) Date of publication of application: **12.02.2025**
(21) Application number: 23781388.6
(22) Date of filing: 30.03.2023
(51) Int. Cl.: C07D 409/12, A61K 31/4184, A61K 31/496, A61K 31/454, A61K 31/541, A61P 35/00, C07D 235/32, C07D 491/113

(54) **THIOBENZIMIDAZOLE DERIVATIVE OR PHARMACEUTICALLY ACCEPTABLE SALT THEREOF, AND USE THEREOF**

(30) Priority: 30.03.2022 KR 20220039893; 29.03.2023 KR 20230041000
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08307 (KR); KIM, Yoon Jae, Paju-si Gyeonggi-do 10907 (KR); KANG, Yong Koo, Seoul 08281 (KR)
(74) Representative: Weickmann & Weickmann PartmbB
(86) International application number: PCT/KR2023/004258
(87) International publication number: WO 2023/191536

(57) **Abstract**

The present invention relates to a thiobenzimidazole derivative or a pharmaceutically acceptable salt thereof, and a composition for preventing or treating cancer, the composition comprising the derivative as an active ingredient. The thiobenzimidazole derivative according to the present invention inhibits tubulin polymerization by being activated in cancer cells, and exhibits cytotoxicity by blocking the cell cycle of cancer cells and inducing apoptosis when administered to an individual, and thus can be used for preventing or treating cancer and preferably, for preventing or treating HER-2 positive breast cancer or triple-negative breast cancer.

## Description

### TECHNICAL FIELD

The present disclosure relates to a thiobenzimidazole derivative or a pharmaceutically acceptable salt thereof, a use thereof, and the like.

### BACKGROUND ART

Patients with triple-negative breast cancer (TNBC; ER-, PR-, HER2-), who account for 10-15% of all breast cancer patients, lack hormone receptors (ER (estrogen receptors), PR (progesterone receptors)) and HER2 proteins, and thus they do not benefit from hormone therapy or HER2-targeted therapy. Currently, the standard of care for triple-negative breast cancer relies entirely on general cytotoxic anti-cancer drugs (Taxene- or anthracene-based agents). Since there are no established targeted therapeutic agents, treatment strategies for other subtypes are less diverse than those for breast cancer. More seriously, after surgery or anticancer treatment, recurrence occurs within 2-3 years in most patients, and metastasis to other organs such as the lungs, liver, brain and bones is easily triggered, affecting the survival rate of the patients.

The 5-year overall survival rate for patients diagnosed with stage III cancer is less than 55%, and for patients whose cancer has already metastasized (advanced-stage), the 5-year overall survival rate is even lower, at 30% or less. This is a very serious disease that will ultimately kill most of these patients within a few years.

Microtubules are a major component of the cytoskeleton and are made up of tubulin heteropolymers, which are composed of α and β subunits. Microtubules perform a variety of cellular functions, including intracellular transport, polarity maintenance, intracellular signaling, and cell migration and proliferation. During cell mitosis, spindle fibers are formed to arrange chromosomes at the center of the cell and then separate them into two poles. If the spindle fibers fail to function properly, cell division is inhibited and apoptosis occurs, making the microtubule an attractive target for anti-cancer drugs.

Drugs targeting microtubules are largely divided into two groups: those that stabilize microtubules and those that destabilize microtubules. First, microtubule stabilizers include taxane, paclitaxel (Taxol), decetaxel, and the like, which serve to prevent microtubules from depolymerization and to enhance polymerization. Most of the microtubule stabilizers bind to either the taxane-binding site or the overlapping site of β-tubulin. Second, microtubule destabilizers include colchicines, vinca alkaloids, and the like, which bind to the colchicine binding site or the vinca binding site. Drugs targeting microtubules themselves are more effective at lower concentrations than drugs affecting microtubule polymers, and the result is the same: they inhibit cell mitosis. Therefore, there is a need to develop a tubulin polymerization inhibitor with potential as an anticancer agent.

On the other hand, flubendazole and albendazole, which are also tubulin polymerization inhibitors, have been commercially available as anthelmintic drugs, but they have been attracting attention as a treatment for various cancers because they may cause cell death by cell cycle arrest, and may also have a killing effect on slow-growing cancer cells. However, there is a limitation that in vivo absorption is difficult due to their low solubility in water, and side effects such as elevated liver enzyme levels appear when they are consumed in large amounts, so the development of derivatives with new structures is required.

Previously known thiosemicarbazones containing a benzimidazole moiety are described in HD Patel et al., Indian Journal of Chemistry, Vol. 52B, April 2013, pp. 535-545, and others.

Accordingly, the present inventors confirmed that a novel thiobenzimidazole derivative or a pharmaceutically acceptable salt thereof induces apoptosis by stopping the cell cycle of cancer cells (cell cycle arrest) as a tubulin polymerization inhibitor, and created the present disclosure.

### DISCLOSURE OF THE INVENTION

### TECHNICAL GOALS

A technical goal to be achieved by the present disclosure is to provide a thiobenzimidazole derivative or a pharmaceutically acceptable salt thereof.

Another technical goal to be achieved by the present disclosure is to provide a method for producing the thiobenzimidazole derivative or the pharmaceutically acceptable salt thereof.

In addition, another goal of the present disclosure is to provide a pharmaceutical composition for preventing or treating a cancer, including the thiobenzimidazole derivative or the pharmaceutically acceptable salt thereof as an active ingredient.

However, the technical goals to be achieved by the present disclosure are not limited to those mentioned above, and other goals not mentioned will be clearly understood by one of ordinary skill in the art from the following description.

### TECHNICAL SOLUTIONS

In order to achieve the above mentioned goals, the present disclosure provides a thiobenzimidazole derivative represented by the following [Chemical Formula 1], a racemate, an isomer, a solvate, or a pharmaceutically acceptable salt thereof:

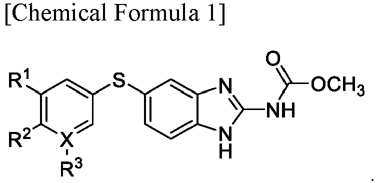

In Chemical Formula 1,
X is any one selected from C or N,
R¹, R² and R³ are each independently any one selected from the group consisting of hydrogen, halogen, -OR⁴, -NR⁵R⁶, a substituted or unsubstituted C₃ to C₂₀ cycloalkyl group, or a substituted or unsubstituted C₃ to C₂₀ heterocycloalkyl group, a substituted or unsubstituted C₃ to C₂₀ aryl group, a substituted or unsubstituted C₃ to C₂₀ heteroaryl group, and a substituted or unsubstituted sulfonamide group,
R⁴ is an alkyl group of C₁-C₁₂,
R⁵ and R⁶ are each independently any one selected from the group consisting of hydrogen, a C₁-C₁₂ alkyl group, -(CH₂)ₙ-N(R⁴)₂, a substituted or unsubstituted C₃ to C₂₀ aryl group, and a substituted or unsubstituted C₃ to C₂₀ heteroaryl group,
n is an integer from "0" to "6."
In an embodiment of the present disclosure, in [Chemical Formula 1],
a substituted cycloalkyl group, heterocycloalkyl group, aryl group, heteroaryl group or sulfonamide group
may be substituted with one or more selected from the group consisting of a substituted or unsubstituted C₁-C₆ alkyl group, a substituted or unsubstituted sulfonyl group, a substituted or unsubstituted carbonyl group, a substituted or unsubstituted hydroxy group, and a heteroaryl group.

In another embodiment of the present disclosure, the thiobenzimidazole derivative represented by [Chemical Formula 1] may be any one selected from the group consisting of the following compounds: 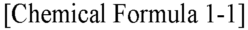 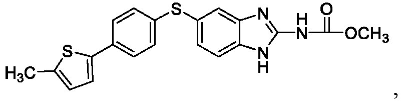 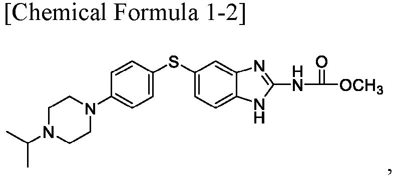 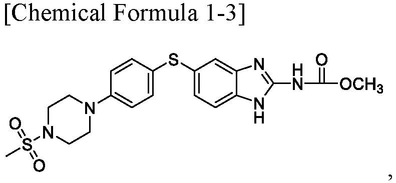 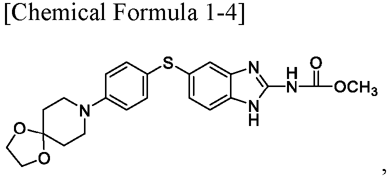 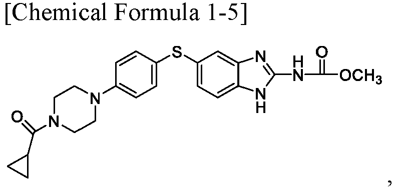 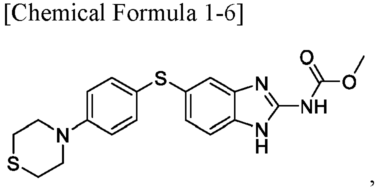 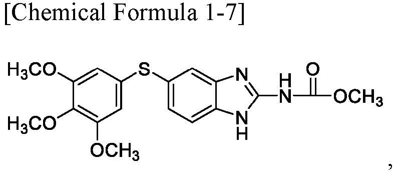 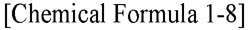 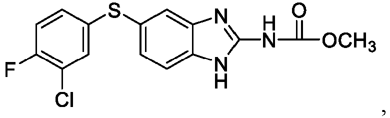 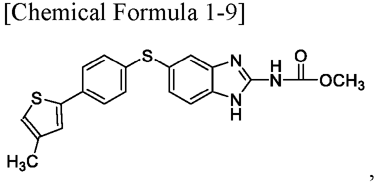 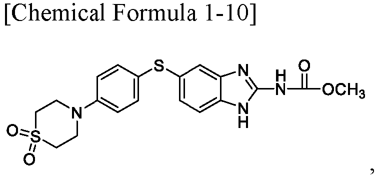 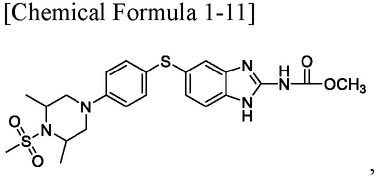 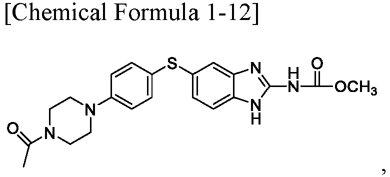 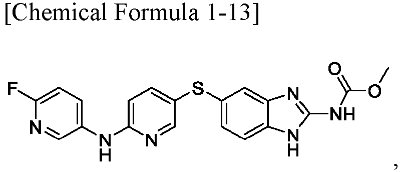 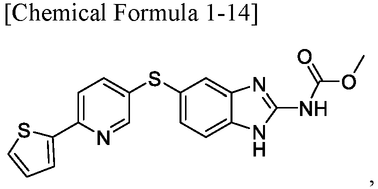 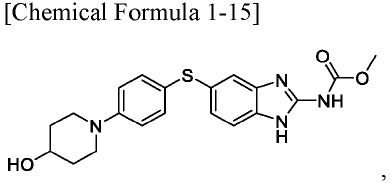 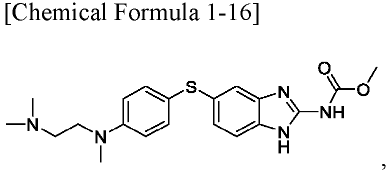 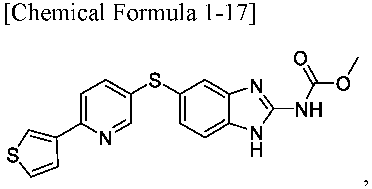 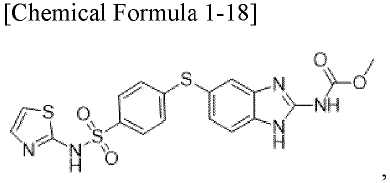 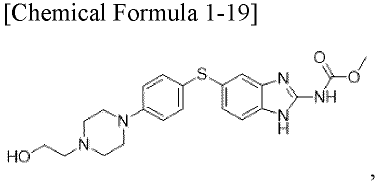 and

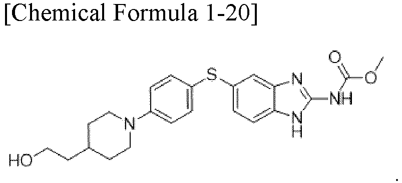

In another embodiment of the present disclosure, the thiobenzimidazole derivative may inhibit tubulin polymerization.

In another embodiment of the present disclosure, the pharmaceutically acceptable salt of the thiobenzimidazole derivative may be one or more selected from the group consisting of hydrochloride, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, paratoluenesulfonate, camphorsulfonate, sodium salt, potassium salt, lithium salt, calcium salt, and magnesium salt, and preferably may be a hydrochloride salt.

Additionally, the present disclosure provides a composition for preventing or treating a cancer, including the thiobenzimidazole derivative or a pharmaceutically acceptable salt thereof as an active ingredient.

In addition, the present disclosure provides a method for preventing or treating a cancer, including administering the thiobenzimidazole derivative or a pharmaceutically acceptable salt thereof to an individual.

Furthermore, the present disclosure provides a use of the thiobenzimidazole derivative or a pharmaceutically acceptable salt thereof for manufacturing a medicament for preventing or treating a cancer.

Also, the present disclosure provides a method for diagnosing a cancer, including administering the thiobenzimidazole derivative or a pharmaceutically acceptable salt thereof to an individual.

Additionally, the present disclosure provides a use of the thiobenzimidazole derivative or a pharmaceutically acceptable salt thereof for manufacturing a medicament for diagnosing a cancer.

In an embodiment of the present disclosure, the pharmaceutical composition may induce apoptosis by arresting a cell cycle of a cancer cell.

In another embodiment of the present disclosure, the cancer may be any one or more selected from the group consisting of skin cancer, breast cancer, uterine cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, and bronchial cancer, preferably breast cancer, more preferably HER-2 positive breast cancer or triple-negative breast cancer.

### EFFECTS OF THE INVENTION

The present disclosure relates to a thiobenzimidazole derivative or a pharmaceutically acceptable salt thereof, and a composition for preventing or treating cancer, the composition including the derivative as an active ingredient. The thiobenzimidazole derivative according to the present disclosure inhibits tubulin polymerization by being activated in cancer cells, and exhibits cytotoxicity by blocking the cell cycle of cancer cells and inducing apoptosis when administered to an individual, and thus may be used for preventing or treating cancer, and preferably, for preventing or treating HER-2 positive breast cancer or triple-negative breast cancer.

The effects of the present disclosure are not limited to those mentioned above, and other effects not mentioned may be clearly understood by one of ordinary skill in the art from the following descriptions.

### BRIEF DESCRIPTION OF THE DRAWINGS

FIG. 1 shows a cell viability of an MDA-MB-231 cell line when treated with a thiobenzimidazole derivative of the present disclosure.
FIG. 2 shows a cell viability of a JIMT-1 cell line when treated with a thiobenzimidazole derivative of the present disclosure.
FIG. 3 shows a cell viability and IC₅₀ values after MDA-MB-231 and JIMT-1 cell lines have been treated with the thiobenzimidazole derivatives of the present disclosure at different concentrations.

### BEST MODE FOR CARRYING OUT THE INVENTION

As a result of intensive research on a thiobenzimidazole derivative or a pharmaceutically acceptable salt thereof, the present inventors confirmed the anti-cancer activity of the thiobenzimidazole derivative and then created the present disclosure.

More specifically, the present inventors confirmed that the thiobenzimidazole derivative or a pharmaceutically acceptable salt thereof is being activated in cancer cells and exhibits cytotoxicity by inhibiting tubulin polymerization, thereby blocking the cell cycle of a cancer cell and inducing apoptosis.

As shown by the above mentioned results, the present disclosure may provide a thiobenzimidazole derivative or a pharmaceutically acceptable salt thereof represented by the following [Chemical Formula 1]:

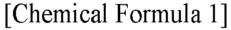

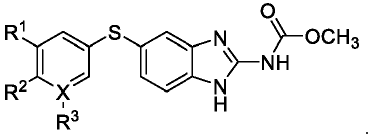

In Chemical Formula 1,
X is any one selected from C or N,
R¹, R² and R³ are each independently any one selected from the group consisting of hydrogen, halogen, -OR⁴, -NR⁵R⁶, a substituted or unsubstituted C₃ to C₂₀ cycloalkyl group, or a substituted or unsubstituted C₃ to C₂₀ heterocycloalkyl group, a substituted or unsubstituted C₃ to C₂₀ aryl group, a substituted or unsubstituted C₃ to C₂₀ heteroaryl group, and a substituted or unsubstituted sulfonamide group,
R⁴ is an alkyl group of C₁-C₁₂,
R⁵ and R⁶ are each independently any one selected from the group consisting of hydrogen, a C₁-C₁₂ alkyl group, -(CH₂)ₙ-N(R⁴)₂, a substituted or unsubstituted C₃ to C₂₀ aryl group, and a substituted or unsubstituted C₃ to C₂₀ heteroaryl group,
n is an integer from "0" to "6."

As used herein, the term "substitution" refers to a reaction in which an atom or atomic group contained in a molecule of a compound is replaced with another atom or atomic group.

As used herein, the term "chained" refers to a molecule with a chain structure, and the chained structure is a chemical structure in which carbon atoms are connected in a chain shape, and there are two types: straight chain and branched chain.

As used herein, the term "cyclo or cyclic" refers to a structure in which the backbone of an organic compound has two ends that are connected to form a ring.

As used herein, the term "chained or cyclic alkyl group" means a monovalent linear or branched or cyclic saturated hydrocarbon residue having 1 to 20 carbon atoms and consisting only of carbon and hydrogen atoms. Examples of such alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl, etc.

As used herein, the term "heterocycloalkyl group" typically refers to a saturated or unsaturated (but not aromatic) cyclohydrocarbon, which may be optionally unsubstituted, mono-substituted or poly-substituted, wherein at least one of the heteroatoms in the structure is selected from N, O or S.

As used herein, the term "aryl group" means an unsaturated aromatic ring compound having 3 to 12 carbon atoms having a single ring (e.g., phenyl) or a plurality of condensed rings (e.g., naphthyl). Examples of such aryl groups include, but are not limited to, phenyl, naphthyl, etc.

As used herein, the term "heteroaryl group" refers to a single ring or a plurality of condensed rings in which at least one of the atoms constituting the ring is a heteroatom of N, O, or S. Examples of such heteroaryl groups include, but are not limited to, pyridyl groups, pyrimidinyl groups, pyrazinyl groups, oxazolyl groups, furyl groups, thiophenyl groups, etc.

As used herein, the term "halogen group" may refer to fluorine (F), chloride (Cl), bromine (Br), or iodine (I), etc.

As used herein, the term "carbonyl group" means -C(=O)- group, the term "sulfonyl group" means -S(=O)₂- group, and the term "sulfonamide group" means -S(=O)₂-NH- group.

As used herein, more specifically, each of the R¹, R² and R³ may independently be hydrogen, fluorine, chlorine, -OCH₃,

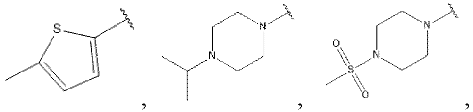

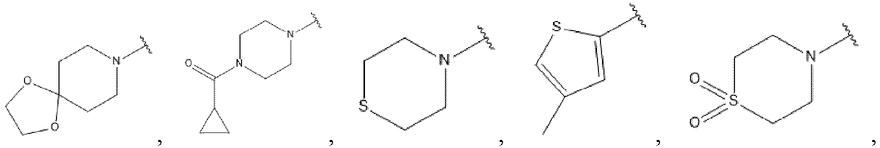

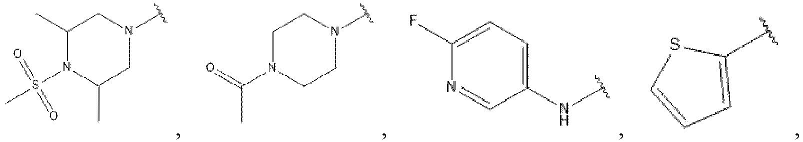

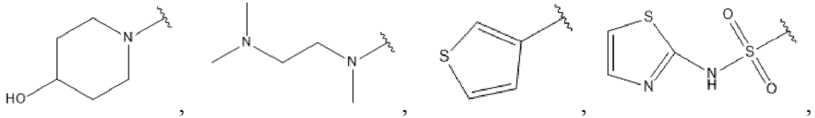

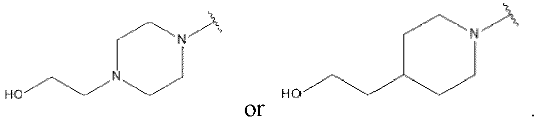

As a preferred embodiment of the present disclosure, the compound represented by Chemical Formula 1 is preferably one or more selected from the group consisting of the following compounds: 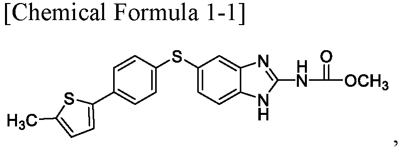 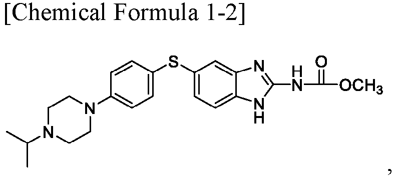 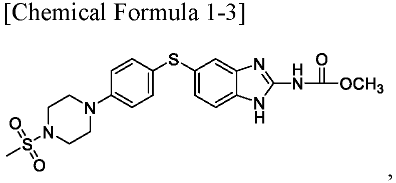 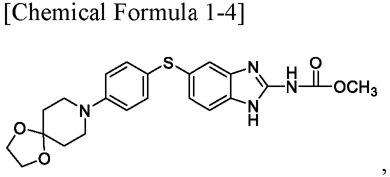 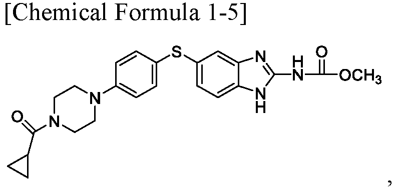 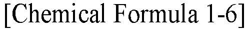 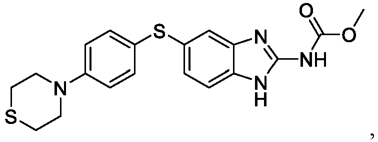 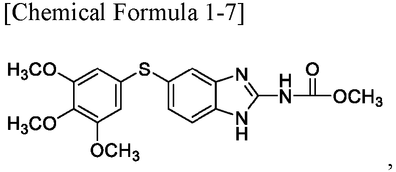 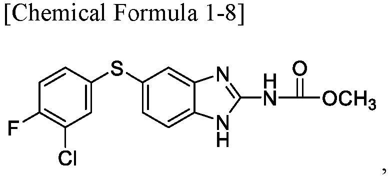 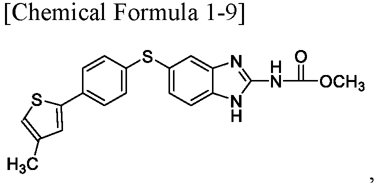 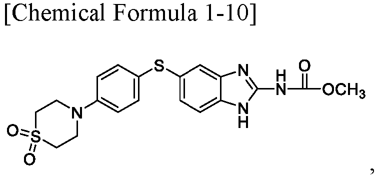 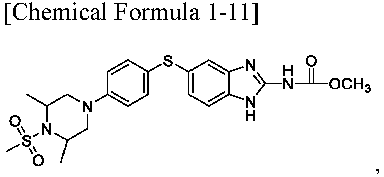 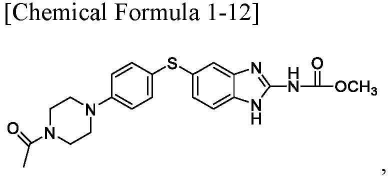 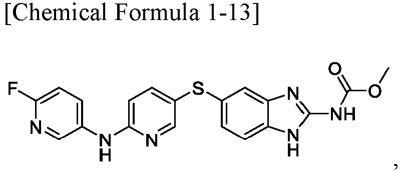 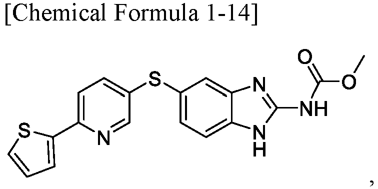 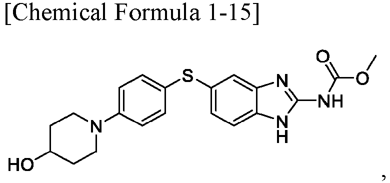 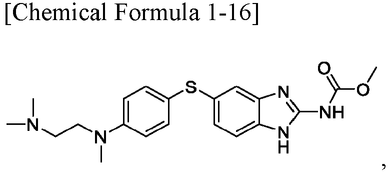 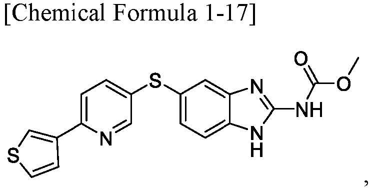 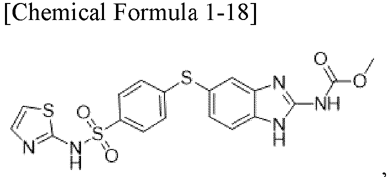 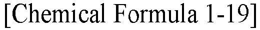 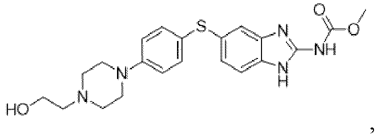 and

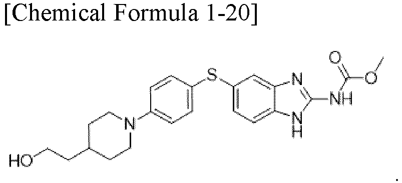

The thiobenzimidazole derivative or pharmaceutically acceptable salt thereof of the present disclosure is activated specifically for cancer cells, thereby inhibiting tubulin polymerisation and inducing cell death, and thus may be used in the pharmaceutical compositions for preventing or treating cancer including the thiobenzimidazole derivative or pharmaceutically acceptable salt thereof as an active ingredient.

As used herein, the term "pharmaceutically acceptable salt" means a formulation of a compound that does not cause serious irritation to the organism to which the compound is administered and does not impair the biological activity and properties of the compound. The pharmaceutically acceptable salt of the compound of the present disclosure may be obtained by reacting the compound of the present disclosure with inorganic acids such as hydrochloric acid, hydrobromic acid, sulfuric acid, nitric acid, and phosphoric acid, sulfonic acids such as methanesulfonic acid, ethanesulfonic acid, and p-toluenesulfonic acid, organic carboxylic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. Also, the pharmaceutically acceptable salt of the compound of the present disclosure may be obtained by reacting the compound of the present disclosure with a base to form salts such as ammonium salt, alkali metal salt such as sodium or potassium salt, alkaline earth metal salt such as calcium or magnesium salt, salts of organic bases such as dicyclohexylamine, N-methyl-D-glucamine, tris(hydroxymethyl)methylamine and salts of amino acids such as arginine and lysine.

Furthermore, the thiobenzimidazole derivative or the pharmaceutically acceptable salt thereof may include not only pharmaceutically acceptable salts, but also all salts, hydrates and solvates that may be prepared by conventional methods.

In addition, the present disclosure provides a method for preventing, treating and/or diagnosing a cancer, including administering the thiobenzimidazole derivative or a pharmaceutically acceptable salt thereof to an individual.

As used herein, the term "prevention" refers to all actions that inhibit or delay the occurrence, spread, or recurrence of cancer by the administration of the composition of the present disclosure, and the term "treatment" refers to all actions by which the symptoms of the disease are ameliorated or beneficially changed by the administration of a composition of the present disclosure.

As used herein, the term "pharmaceutical composition" means a composition formulated for the prevention or treatment of a disease, each of which may be formulated in various forms according to a conventional method and used. For example, the composition may be formulated in oral dosage forms such as powders, granules, tablets, capsules, suspensions, emulsions, syrups, and the like, and may be formulated in the form of external preparations, suppositories, and sterile injectable solutions and used.

As used herein, the phrase "including as an active ingredient" means that the ingredient is included in an amount necessary or sufficient to realize a desired biological effect. In practical application, the amount to be included as an active ingredient may be determined to be an amount to treat the disease of interest and not cause other toxicities. For example, the amount may vary depending on a variety of factors, such as the disease or condition being treated, the form of the composition being administered, the size of the individual, or the severity of the disease or condition. One of ordinary skill in the art to which the present disclosure pertains will be able to determine empirically the effective amount of an individual composition without an undue amount of experimentation.

In addition, the pharmaceutical composition of the present disclosure may include one or more pharmaceutically acceptable carriers in addition to the active ingredients described above, depending on the formulation.

The pharmaceutically acceptable carrier may be saline, sterile water, Ringer's solution, buffered saline, dextrose solution, maltodextrin solution, glycerol, ethanol, and a mixture of one or more of these components, and may further include other conventional additives, such as antioxidants, buffers, bacteriostatic agents, and the like. In addition, the composition may be formulated in injectable forms, such as aqueous solutions, suspensions, emulsions, etc., pills, capsules, granules or tablets by additionally adding diluents, dispersants, surfactants, binders and lubricants. Further, the composition may be formulated by any suitable method in the art, or as disclosed in Remington's Pharmaceutical Science (Mack Publishing Company, Easton PA), depending on the disease or ingredient.

The compositions of the present disclosure may be administered orally or parenterally in a pharmaceutically effective amount, depending on the intended method, and the term "pharmaceutically effective amount" as used herein means an amount sufficient to treat a disease where a benefit/risk ratio applicable to medical treatment is reasonable and not cause adverse effects. The effective dose level may be determined based on factors including the patient's health condition, severity, activity of the drug, sensitivity to the drug, method of administration, time of administration, route of administration and rate of elimination, duration of treatment, drugs used in combination or concurrently, and other factors well known in the medical field.

Therefore, the pharmaceutical composition of the present disclosure may be administered to an individual to prevent, treat, and/or diagnose cancer, where the cancer may be skin cancer, breast cancer, uterine cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, bronchial cancer, etc., and preferably may be, but is not limited to, cancers that have a higher acidity than normal cells and whose cytotoxicity is inhibited by tubulin polymerization inhibitors, such as, but not limited to, breast cancer, preferably HER-2 positive breast cancer or triple-negative breast cancer.

As used herein, the term "individual" may refer to, but is not limited to, any mammal, such as a domestic animal or human, in need of prevention, treatment, and/or diagnosis of a cancer, preferably a human.

The pharmaceutical composition of the present disclosure may be formulated in various forms for administration to an individual, and the most representative of the forms for parenteral administration is an injectable form, preferably an isotonic aqueous solution or suspension. The injectable form may be prepared according to techniques known in the art using a suitable dispersing or wetting agent and a suspending agent. For example, each component may be dissolved in saline or a buffer solution and the mixture may be formulated for injection. In addition, the forms for oral administration include, for example, swallowable tablets, buccal tablets, troches, capsules, elixirs, suspensions, syrups, and wafers, which may include, in addition to an active ingredient, a diluent (e.g., lactose, dextrose, sucrose, mannitol, sorbitol, cellulose, and/or glycine) and an active agent (e.g., silica, talc, stearic acid and its magnesium or calcium salt, and/or polyethylene glycol). The tablets may include a binder such as magnesium aluminum silicate, starch paste, gelatin, tragacanth, methylcellulose, sodium carboxymethylcellulose, and/or polyvinylpyrrolidine, and in some cases may further include disintegrating agents such as starch, agar, alginic acid or its sodium salts, absorbents, colorants, flavoring agents, and/or sweeteners. The formulations may be prepared by conventional mixing, granulating, or coating methods.

In addition, the pharmaceutical composition of the present disclosure may further contain adjuvants such as preservatives, hydrating agents, emulsification accelerators, salts or buffers for osmotic pressure control, and other therapeutically useful substances, and may be formulated according to a conventional method.

The pharmaceutical composition according to the present disclosure may be administered via several routes, including oral, transdermal, subcutaneous, intravenous, and intramuscular, and the dosage of the active ingredient may be adequately selected depending on several factors, including the route of administration, the age, gender, and weight of the patient, and the severity of the patient's disease. In addition, the composition of the present disclosure may be administered in combination with the known compounds, which may enhance the desired effect.

The routes of administration of the pharmaceutical composition according to the present disclosure can be administered to humans and animals orally or parenterally, for example, intravenously, subcutaneously, intranasally or intraperitoneally. Oral administration also includes sublingual application. Parenteral administration includes injection and drop methods such as subcutaneous injection, intramuscular injection, and intravenous injection.

In the pharmaceutical composition of the present disclosure, the total effective doses of a thiobenzimidazole derivative or a pharmaceutically acceptable salt thereof according to the present disclosure may be administered to a patient in a single dose, or following a fractionated treatment protocol according to which multiple doses are administered over a longer period of time. The pharmaceutical composition of the present disclosure may vary in the amount of active ingredients depending on the severity of the disease. Typically in an adult, the composition may be administered multiple times per day at an effective dose of 100 µg to 3,000 mg per dose. However, the concentration of the thiobenzimidazole derivative or the pharmaceutically acceptable salt thereof may vary depending on the route of administration and the number of treatments. Further, the effective dose for the patient may be determined considering various factors such as the patient's age, weight, health status, and sex, the severity of the disease, and the patient's diet and excretion rate.

In addition, the pharmaceutical composition according to the present disclosure is not particularly limited in terms of formulation, route of administration and method of administration, so long as it exhibits the effects of the present disclosure, and may additionally comprise known anti-cancer agents in addition to the above mentioned thiobenzimidazole derivatives or pharmaceutically acceptable salts thereof as active ingredients, and may also be used in combination with other known therapies for the treatment of these diseases.

The terminology used in embodiments is for the purpose of describing particular embodiments only and is not intended to be limiting. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, components, and/or groups thereof but do not preclude the presence or addition of one or more other features, integers, steps, operations, elements, components, and/or groups thereof.

Unless otherwise defined, all terms including technical or scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

As the inventive concept allows for various changes and numerous embodiments, particular embodiments will be illustrated in the drawings and described in detail in the written description. However, such illustrations and descriptions are not intended to limit the present disclosure to specific embodiments, and should be understood as including all changes, equivalents, and replacements within the idea and the technical scope of the present disclosure. In the description of embodiments, detailed description of well-known related structures or functions will be omitted when it is deemed that such description will cause ambiguous interpretation of the present disclosure.

Hereinafter, embodiments will be described in detail with reference to the accompanying drawings. However, since various changes may be made to the embodiments, it will be understood that the descriptions of the embodiments do not limit or otherwise restrict the scope of the patent application. With respect to the embodiments, it should be understood that the scope of the rights shall include all modifications, equivalents, and replacements.

### MODE FOR CARRYING OUT THE INVENTION

### Preparation Example 1. Synthesis of Chemical Formula 1-1

The synthesis process of the thiobenzimidazole derivative of Chemical Formula 1-1 is shown by Reaction Scheme 1 presented below.

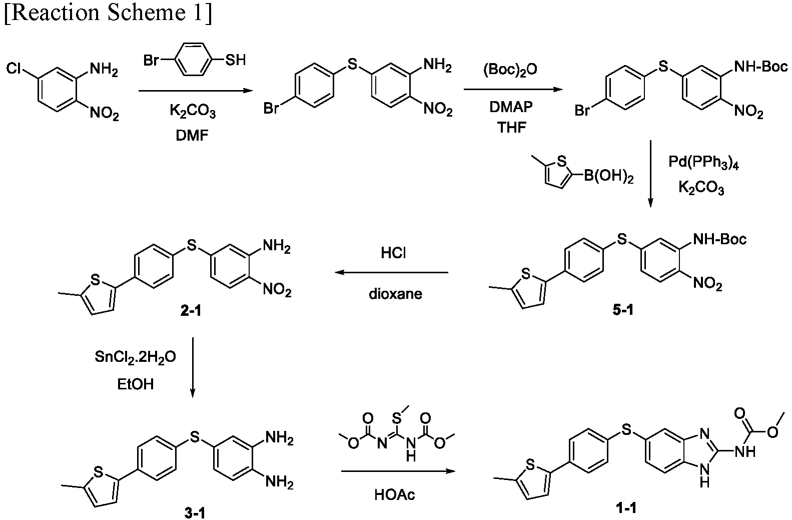

### 1.1. Synthesis of 5-((4-(5-methylthiophen-2-yl)phenyl)thio)-2-nitroaniline (Chemical Formula 2-1)

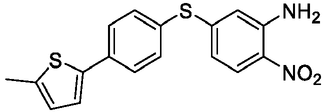

### (1) Synthesis of 5-((4-Bromophenyl)thio)-2-nitroaniline

In a solution of 4-bromobenzenethiol (1.4 g, 8.7 mmol) in DMF (10 mL) was dissolved potassium carbonate (1.24 g). To the mixture was added a solution of 5-chloro-2-nitroaniline (1.49 g, 8.7 mmol) in DMF (3 mL) previously prepared at room temperature. The reaction mixture was stirred at 90°C for 3 hours, cooled to room temperature, poured into cold water (500 mL), and the precipitate was filtered and dried to obtain 5-((4-bromophenyl)thio)-2-nitroaniline (2.8 g) as a yellow solid (yield 99%).

### (2) Synthesis of tert-butyl (5-((4-bromophenyl)thio)-2-nitrophenyl)carbamate

To a solution of 5-((4-bromophenyl)thio)-2-nitroaniline (2.8 g, 8.7 mmol) in tetrahydrofuran (THF, 30 mL) were added Di-tert-butyl dicarbonate (5.14 g, 23.5 mmol) and DMAP (0.146 g), and the mixture was heated and refluxed for 1 hour to react. The completion of the reaction was confirmed by TLC, then the reaction mixture was cooled to room temperature and evaporated under reduced pressure to remove the solvent. The product was dissolved in methanol (30 mL) and potassium carbonate (5.2 g) was added. The mixture was stirred at room temperature for 6 hours, and then was treated to obtain tert-butyl (5-((4-bromophenyl)thio)-2-nitrophenyl)carbamate (1.48 g) (yield 40%).

### (3) Synthesis of tert-butyl (2-nitro-5-((4-(thiophen-2-yl)phenyl)thio)phenyl)carbamate (Chemical Formula 5-1)

To a solution of tert-butyl (5-((4-bromophenyl)thio)-2-nitrophenyl)carbamate (0.7 g, 1.6 mmol) in DMF (5 mL) were added (5-methylthiophen-2-yl)boronic acid (0.466 g, 3.28 mmol, 2.0 eq), Tetrakis(triphenylphosphine)palladium (Pd(PPh₃)₄, 0.18 g, 0.16 mmol, 0.1 eq), and potassium carbonate (K₂CO₃, 0.66 g, 4.80 mmol, 3 eq), respectively. The reaction mixture was stirred at 100°C for 10 hours. The reaction mixture was cooled to room temperature, extracted with methylene chloride solution, and the extract solution was washed once with brine solution, dried over anhydrous magnesium sulfate (MgSO₄), evaporated under reduced pressure, and then separated and purified by Silica-Gel column chromatography with ethyl acetate and hexane (7:3, v/v) eluent to obtain tert-butyl (5-((4-(5-methylthiophen-2-yl)phenyl)thio)-2-nitrophenyl)carbamate (Chemical Formula **5-1,** 0.501 g) as a light yellow solid (yield 67%).

### (4) Synthesis of 5-((4-(5-methylthiophen-2-yl)phenyl)thio)-2-nitroaniline (Chemical Formula 2-1)

A solution of tert-butyl (5-((4-(5-methylthiophen-2-yl)phenyl)thio)-2-nitrophenyl)carbamate (Chemical Formula **5-1,** 0.486 g, 1.1 mmol) in 4 moles of hydrogen chloride/dioxane (HCl, 6 mL) was reacted at room temperature for 1 hour, then poured into a cold saturated aqueous solution of sodium bicarbonate (30 mL), and extracted with excess methylene chloride solution. The extract was dried over anhydrous sodium sulfate. The residue was filtered, and the solvent was removed by evaporation under reduced pressure. Then, the residue was purified by silica gel column chromatography to obtain 5-((4-(5-methylthiophen-2-yl)phenyl)thio)-2-nitroaniline (Chemical Formula **2-1,** 271 mg) (yield 72%).
¹H NMR (CDCl₃, 400 MHz) δ 8.02(s, 1H), 7.64(d, J=8.8Hz, 2H), 7.53(d, J=8.4Jz, 2H), 7.22(s, 1H), 6.79(s, 1H), 6.49(d, J=9.2Hz, 1H), 6.38(s, 1H), 6.05(m, 2H), 2.55(s, 3H)

### 1.2. Synthesis of 4-((4-(5-methylthiophen-2-yl)phenyl)thio)benzene-1,2-diamine (Chemical Formula 3-1)

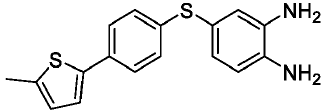

To a solution of 5-((4-(5-methylthiophen-2-yl)phenyl)thio)-2-nitroaniline (Chemical Formula **2-1, 270** mg, 0.79 mmol) in ethanol (10 mL) was added tin(II) chloride dihydrate (SnCl₂.2H₂O, 1.14 g, 5.04 mmol, 6.4 eq). The mixture was heated and refluxed for 3 hours, then cooled to room temperature. Water (35 mL) was added. Extraction was performed with excess ethyl acetate solvent. The extract was dried over anhydrous magnesium sulfate, and the solvent was removed by evaporation under reduced pressure to obtain a solid-phase product. The product was separated and purified by silica gel column chromatography using a solvent of ethyl acetate and hexane (1:3, v/v) to obtain 4-((4-(5-methylthiophen-2-yl)phenyl)thio)benzene-1,2-diamine (Chemical Formula **3-1,** 0.148 g) (yield 60%).
¹H NMR (CDCl₃, 400 MHz) δ 7.58(d, J=8.8Hz, 2H), 7.53(d, J=8.8Jz, 2H), 7.23(s, 1H), 6.83 - 6.49 (m, 4H), 2.45(s, 3H)

### 1.3. Synthesis of methyl (5-((4-(5-methylthiophen-2-yl)phenyl)thio)-1H-benzo[d]imidazol-2-yl)carbamate (Chemical Formula 1-1)

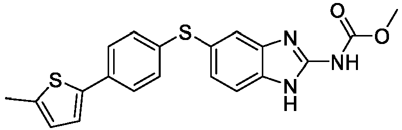

4-((4-(5-methylthiophen-2-yl)phenyl)thio)benzene-1,2-diamine (Chemical Formula **3-1,** 0.103 g, 0.33 mmole) and 1,3-bis(methoxycarbonyl)-S-methylisothiourea (0.55 g, 2.6 mmole, 8 eq) were dissolved in 3 ml of 5%-AcOH in Ethanol and reacted at 80°C for 2 hours. The completion of the reaction was confirmed by TLC. The reaction mixture was extracted with excess methylene chloride. The extract was neutralized with saturated sodium bicarbonate solution, dried over anhydrous sodium sulfate, and separated by silica gel column chromatography to afford methyl (5-((4-(5-methylthiophen-2-yl)phenyl)thio)-1H-benzo[d]imidazol-2-yl)carbamate (Chemical Formula **1-1,** 85 mg) as a light brown solid (yield 65%).
¹H NMR (CDCl₃, 400 MHz) δ 2.21(s, 3H), 3.76(s, 3H), 7.02-7.28(m, 5H), 7.45(d, J=8.8Hz, 2H), 7.53(m, 2H), 11.42(m, 1H), 12.02(m, 1H)

### Preparation Example 2. Synthesis of Chemical Formula 1-2

The synthesis process of the thiobenzimidazole derivative of Chemical Formula 1-2 is shown by Reaction Scheme 2 presented below.

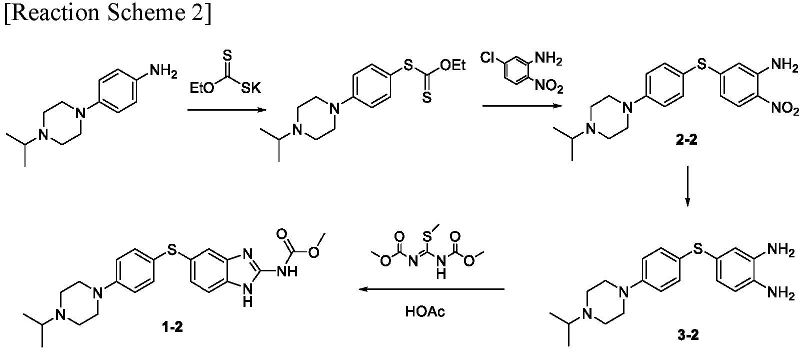

### 2.1. Synthesis of 5-((4-(4-isopropylpiperazin-1-yl)phenyl)thio)-2-nitroaniline (Chemical Formula 2-2)

### (1) Synthesis of O-ethyl S-(4-(4-isopropylpiperazin-1-yl)phenyl)carbonodithioate

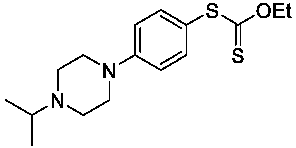

To a mixed solution of 4-[4-(1-methylethyl)-1-piperazinyl]benzenamine (2.19 g, 10 mmol) in THF/H₂O (50 mL/50 mL) was slowly sequentially added dropwise sulfuric acid (1.7 g, 17.3 mmol) and NaNO₂ (1.4 g, 20.4 mmol) dissolved in water (3 mL) in an ice bath at 0-5°C. The reaction mixture was stirred for 30 minutes, potassium O-ethyl carbonodithioate (8.18 g, 51 mmol) was added, and the mixture was stirred at room temperature for 2 hours. After the reaction was completed, the resulting mixture was poured into water (100 mL) and extracted with ethyl acetate. The organic layer was dried over anhydrous sodium sulfate, the solvent was concentrated by evaporation under reduced pressure, and then the residue was separated and purified using reverse-phase column chromatography using acetonitrile/water to afford O-ethyl S-(4-(4-isopropylpiperazin-1-yl)phenyl)carbonodithioate (Chemical Formula **3-3**) (yield 38%).
¹H NMR (CDCl₃, 400 MHz) δ 7.34(d, J=8.8Hz, 2H), 6.94(d, J=8.8Hz, 2H), 4.64(q, 2H), 3.32(m, 4H), 2.70(m, 5H), 1.38(t, 3H), 1.12(d, J=6.4Hz, 6H)

### (2) Synthesis of 5-((4-(4-isopropylpiperazin-1-yl)phenyl)thio)-2-nitroaniline (Chemical Formula 2-2)

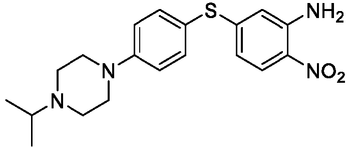

To a solution of O-ethyl S-(4-(4-(4-isopropylpiperazin-1-yl)phenyl) carbonodithioate (1.62 g, 5 mmol) in THF/MeOH (30 mL/15 mL) was added 5-chloro-2-nitroaniline (1.72 g, 10 mmol) and CH₃ONa (5M in MeOH, 4.0 mL, 20.28 mmol), and the reaction mixture was reacted at 80°C for 6 hours. After the reaction was completed, the pH of the resulting mixture was adjusted to pH 7-8 with sodium hydroxide and aqueous acetic acid solution, and the resulting mixture was extracted with methylene chloride. The extract was dried and the solvent was removed. The resulting mixture was separated and purified by silica gel column chromatography using hexane/ethylacetate 4:2, DCM/ MeOH 10:1 to afford 5-((4-(4-isopropylpiperazin-1-yl)phenyl)thio)-2-nitroaniline (Chemical Formula **2-2**) as a light yellow solid (yield 63%).
¹H NMR (CDCl₃, 400 MHz) δ 7.98(d, J=8.8Hz, 1H), 7.44(d, J=8.8Hz, 2H), 6.96(d, J=8.8Hz, 2H), 6.42(m, 1H), 6.26(s, 1H), 6.02(m, 2H), 3.33(m, 4H), 2.78(m, 1H), 2.71(m, 4H), 1.11(d, J=6.4Hz, 6H)

### 2.2. Synthesis of 4-((4-(4-isopropylpiperazin-1-yl)phenyl)thio)benzene-1,2-diamine (Chemical Formula 3-2)

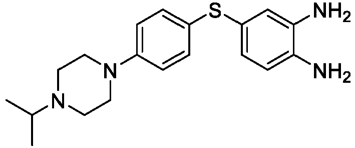

To a solution of 5-((4-(4-(4-isopropylpiperazin-1-yl)phenyl)thio)-2-nitroaniline (Chemical Formula **2-2,** 400 mg, 1.07 mmol) in acetic acid (7 mL) was added activated zinc powder (400 mg), and the reaction mixture was stirred at room temperature for 16 hours. The reaction mixture was filtered, the filtrate was evaporated under reduced pressure to remove excess acetic acid, the pH was slightly alkalized with sodium bicarbonate, then the mixture was extracted with ethyl acetate, and the residue was purified to afford 4-((4-(4-isopropylpiperazin-1-yl)phenyl)thio)benzene-1,2-diamine (Chemical Formula **3-2,** 333 mg) as a yellow solid (yield 91%).
¹H NMR (CDCl₃, 400 MHz) δ 7.33(m, 4H), 7.24(d, J=8.4Hz, 2H), 6.84(d, J=8.4Hz, 2H), 6.74(m, 2H), 6.64(m, 1H), 3.40(m, 4H), 3.34(m, 1H), 3.12(m, 4H), 1.28(d, J=6.4Hz, 6H)

### 2.3. Synthesis of methyl (5-((4-(4-isopropylpiperazin-1-yl)phenyl)thio)-1H-benzo[d]imidazol-2-yl)carbamate (Chemical Formula 1-2)

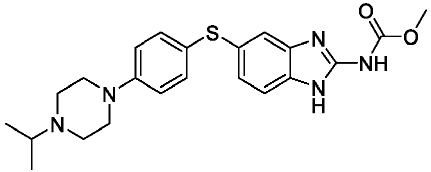

To an solution of 4-((4-(4-(4-isopropylpiperazin-1-yl)phenyl)thio)benzene-1,2-diamine (Chemical Formula **3-2,** 150 mg, 0.44 mmol) in 5% acetic acid ethyl alcohol (3 mL) was added 1,3-bis(methoxycarbonyl)-S-methylisothiourea (300 mg, 1.45 mmol), and the reaction mixture was heated to reflux for 24 hours. The reaction mixture was extracted with ethyl acetate, and then separated and purified by silica gel column chromatography with a mixed solvent of ethyl acetate and hexane to obtain methyl (5-((4-(4-isopropylpiperazin-1-yl)phenyl)thio)-1H-benzo[d]imidazol-2-yl)carbamate (Chemical Formula **1-2,** 110 mg) as a product (yield 59%).
¹H-NMR (DMSO-d₆, 400 MHz) δ 11.74(m, 2H), 7.34(m, 1H), 7.28(s, 1H), 7.22(d, J= 8.8 Hz, 2H), 7.03(m, 1H), 6.92(d, J = 8.8 Hz, 2H), 3.74(s, 3H), 3.12(m, 4H), 2.66(m, 1H), 2.56(m, 4H), 1.00(d, J = 6.4 Hz, 6H)

### Preparation Example 3. Synthesis of Chemical Formula 1-3

The synthesis process of the thiobenzimidazole derivative of Chemical Formula 1-3 is shown by Reaction Scheme 3 presented below.

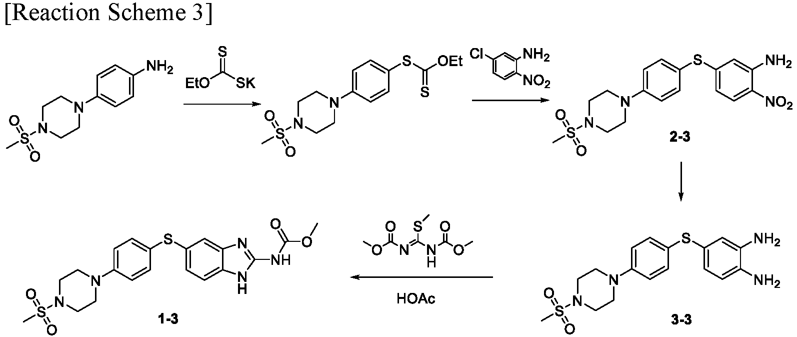

### 3.1. Synthesis of 5-((4-(4-(methylsulfonyl)piperazin-1-yl)phenyl)thio)-2-nitroaniline (Chemical Formula 2-3)

### (1) Synthesis of O-ethyl S-(4-(4-(methylsulfonyl)piperazin-1-yl)phenyl) carbonodithioate

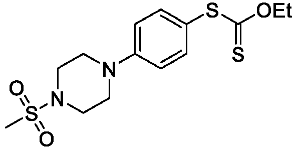

A mixed solution of 4-(4-(methylsulfonyl)piperazin-1-yl)aniline (1.5 g, 5.87 mmol) in THF/MeOH (17/30 mL), sodium nitrite (0.82 g, 11.74 mmol) dissolved in distilled water (6 mL), and concentrated hydrochloric acid (1.0 mL) was prepared at 0°C in an ice bath. To the mixture prepared previously was slowly added dropwise a solution of Potassium Ethylxanthate (6.0 g, 37.4 mmol) in distilled water (15 mL) at 40°C and the mixture was reacted for 1 hour. After the reaction was completed, the mixture was poured into water (150 mL) and extracted with EA (150 mL×2). The organic layer was dried over anhydrous magnesium sulfate, concentrated, and purified with ethyl acetate and n-hexane to obtain O-ethyl S-(4-(4-(methylsulfonyl)piperazin-1-yl)phenyl)carbonodithioate (1.18 g) as a brown solid (yield 56%).
¹H NMR (CDCl₃, 400 MHz) δ 7.42(d, J=8.8Hz, 1H), 7.32(m, 1H), 6.97(m, 2H), 4.65(q, 2H), 3.38(m, 6H), 3.32(m, 2H), 2.86(s, 3H), 1.37(t, 3H)

### (2) Synthesis of 5-((4-(4-(methylsulfonyl)piperazin-1-yl)phenyl)thio)-2-nitroaniline (Chemical Formula 2-3)

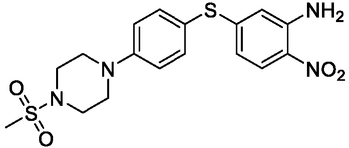

To a solution of O-ethyl S-(4-(4-(methylsulfonyl)piperazin-1-yl)phenyl)carbonodithioate (0.6 g, 1.66 mmol) in THF/MeOH (15/20 mL) was added 5-chloro-2-nitroaniline (0.314 g, 1.82 mmol), then sodium hydroxide powder (0.279 g, 6.97 mmol) dissolved in distilled water (5 mL) was added. The reaction mixture was reacted at 90°C for 4 hours, then separated and purified by a mixed solution of ethyl acetate and hexane to obtain 5-((4-(4-(methylsulfonyl)piperazin-1-yl)phenyl)thio)-2-nitroaniline (Chemical Formula **2-3,** 0.494 g) as a yellow solid (yield 51%).
¹H NMR (CDCl₃, 400 MHz) δ 7.99(d, J=9.2Hz, 1H), 7.48(d, J=8.8Hz, 2H), 7.00(d, J=8.8Hz, 2H), 6.42(m, 1H), 6.30(s, 1H), 6.03(s, 2H), 3.42(s, 8H), 2.87(s, 3H),

### 3.2 Synthesis of 4-((4-(4-(methylsulfonyl)piperazin-1-yl)phenyl)thio)benzene-1,2-diamine (Chemical Formula 3-3)

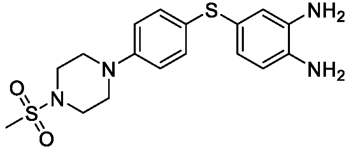

To a solution of 5-((4-(4-(methylsulfonyl)piperazin-1-yl)phenyl)thio)-2-nitroaniline (Chemical Formula **2-3,** 0.330 g, 0.81 mmol) in acetic acid (4 mL) was added activated zinc powder (250 mg) and the mixture was reacted at room temperature for 10 hours. The reaction mixture was filtered, the filtrate was evaporated under reduced pressure to remove excess acetic acid, the pH was slightly alkalized with sodium bicarbonate solution, and then the residue was extracted and purified with ethyl acetate to obtain 4-((4-(4-(methylsulfonyl)piperazin-1-yl)phenyl)thio)benzene-1,2-diamine (Chemical Formula **3-3,** 210 mg) as a yellow solid (yield 69%).
¹H NMR (CDCl₃, 400 MHz) δ 7.25(d, J=8.8Hz, 2H), 6.86(d, J=8.8Hz, 2H), 6.77(m, 2H), 6.67(d, J=8.0Hz, 1H), 3.78(m, 8H), 3.40(m, 4H), 2.84(s, 3H),

### 3.3. Synthesis of methyl (5-((4-(4-(methylsulfonyl)piperazin-1-yl)phenyl)thio)-1H-benzo[d]imidazol-2-yl)carbamate (Chemical Formula 1-3)

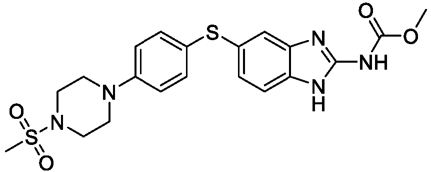

To a solution of 4-((4-(4-(methylsulfonyl)piperazin-1-yl)phenyl)thio)benzene-1,2-diamine (Chemical Formula **3-3,** 140 mg, 0.37 mmol) in 5% acetic acid ethyl alcohol (5 mL), was added 1,3-bis(methoxycarbonyl)-S-methylisothiourea (300 mg, 1.45 mmol) and the mixture was reacted at 110°C for 5 hours. At this time, the insoluble reactants were dissolved. As the reaction time elapsed, a white precipitate was obtained, and methyl alcohol (50 mL) was added at 50°C and the mixture was filtered to obtain the product. The product was further washed with methyl alcohol (100 mL) until the urea odor was removed, to obtain methyl (5-((4-(4-(methylsulfonyl)piperazin-1-yl)phenyl)thio)-1H-benzo[d]imidazol-2-yl)carbamate (Chemical Formula **1-3,** 170 mg) as a pure product (100% yield).
¹H NMR (DMSO-d₆, 400 MHz) δ 11.89(m, 1H), 11.32(m, 1H), 7.36(m, 2H), 7.23(d, J=8.8Hz, 2H), 7.06(m, 1H), 6.98(d, J=8.8Hz, 2H), 3.74(s, 3H), 3.25(m, 8H), 2.92(s, 3H)

### Preparation Example 4. Synthesis of Chemical Formula 1-4

The synthesis process of the thiobenzimidazole derivative of Chemical Formula 1-4 is shown by Reaction Scheme 4 presented below.

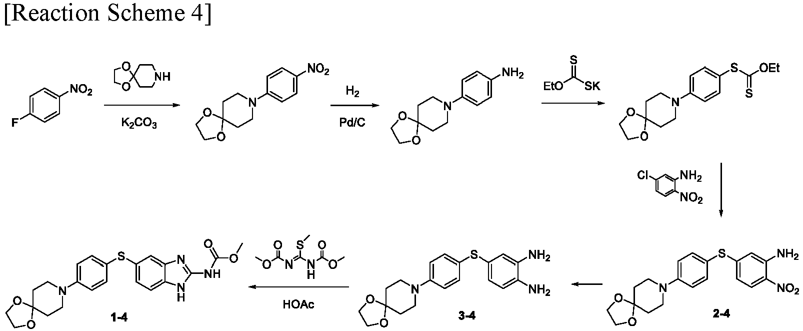

### 4.1. Synthesis of 5-((4-(1-oxa-8-azaspiro[4.5]decan-8-yl)phenyl)thio)-2-nitroaniline (Chemical Formula 2-4)

### (1) Synthesis of 4-(1,4-dioxa-8-azaspiro[4.5]decan-8-yl)aniline

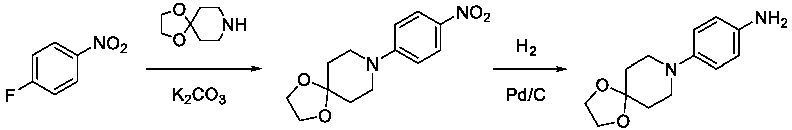

To a solution of 1-fluoro-4-nitrobenzene (1.41 g, 0.01 mol) in DMF (10 mL), were added 1,4-dioxa-8-azaspiro[4.5]decane (2.14 g, 0.015 mol) and potassium carbonate (2.76 g, 0.02 mol) and the mixture was reacted at 100°C for 14 hours. The solid was removed from the reaction mixture at a high temperature, then the residue was cooled to room temperature. The resulting solid precipitate was filtered to obtain 8-(4-nitrophenyl)-1,4-dioxa-8-azaspiro[4.5]decane (2.34 g) as a light yellow solid. 10% of Pd/C (500 mg) was added to the methanol (70 mL) solution of the resulting solid product, hydrogen gas was charged under pressure, and then reaction was performed in a Par reactor for 18 hours. The reaction product was filtered and the filtrate was concentrated to obtain 4-(1,4-dioxa-8-azaspiro[4.5]decan-8-yl)aniline (1.522g) as a light yellow solid (yield 65%).

### (2) Synthesis of S-(4-(1,4-dioxa-8-azaspiro[4.5]decan-8-yl)phenyl) O-ethyl carbonodithioate

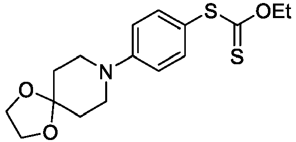

A mixed solution of 4-(1,4-dioxa-8-azaspiro[4.5]decan-8-yl)aniline (1.1 g, 4.69 mmol) in distilled water (15 mL), sodium nitrite (470 mg) dissolved in distilled water (5 mL), and concentrated hydrochloric acid (0.8 mL) was prepared at 0°C in an ice bath. To the mixture prepared previously was slowly added dropwise a solution of Potassium ethylxanthate (1.24 g, 7.48 mmol) in distilled water (3 mL)at 50°C and the mixture was reacted for 1 hour.

After the reaction was completed, the mixture was poured into water (70 mL) and extracted with EA (70 mL×2). The organic layer was dried over anhydrous magnesium sulfate, concentrated, and purified with ethyl acetate and n-hexane to obtain S-(4-(1,4-dioxa-8-azaspiro[4.5]decan-8-yl)phenyl) O-ethyl carbonodithioate (1.11 g) as a brown solid (yield 68%).
¹H NMR (CDCl₃, 400 MHz) δ 7.36(d, J=8.8Hz, 2H), 6.95(d, J=8.8Hz, 2H), 4.64(q, 2H), 3.45(m, 4H), 1.84(m, 4H),1.36(t, 3H)

### (3) Synthesis of 5-((4-(1-oxa-8-azaspiro[4.5]decan-8-yl)phenyl)thio)-2-nitroaniline (Chemical Formula 2-4)

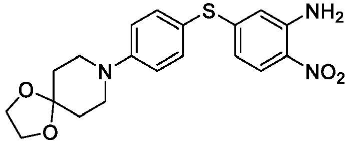

To a solution of S-(4-(1,4-dioxa-8-azaspiro[4.5]decan-8-yl)phenyl) O-ethyl carbonodithioate (770 mg, 2.27 mmol) in THF/MeOH (23/38 mL) was added 5-chloro-2-nitroaniline (400 mg, 3.31 mmol), then sodium hydroxide powder (340 mg) dissolved in distilled water (7.6 mL) was added. The reaction mixture was reacted at 90°C for 5 hours, then separated and purified by a mixed solution of ethyl acetate and hexane to obtain 5-((4-(1-oxa-8-azaspiro[4.5]decan-8-yl)phenyl)thio)-2-nitroaniline (Chemical Formula **2-4,** 470 mg) as a yellow solid (yield 54%).
¹H NMR (CDCl₃, 400 MHz) δ 7.98(d, J=9.2Hz, 1H), 7.42(d, J=8.8Hz, 2H), 6.97(d, J=8.8Hz, 2H), 6.42(m, 1H), 6.25(s, 1H), 6.03(s, 2H), 4.15(s, 4H), 3.47(m, 4H), 1.86(m, 4H),

### 4.2. Synthesis of 4-((4-(1,4-dioxa-8-azaspiro[4.5]decan-8-yl)phenyl)thio)benzene-1,2-diamine (Chemical Formula 3-4)

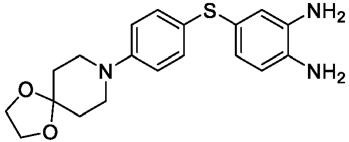

To a solution of 5-((4-(1-oxa-8-azaspiro[4.5]decan-8-yl)phenyl)thio)-2-nitroaniline (Chemical Formula **2-4,** 450 mg, 1.17 mmol) in acetic acid (7 mL), was added activated zinc powder (450 mg) and the mixture was reacted at room temperature for 7 hours. The reaction mixture was filtered, the filtrate was evaporated under reduced pressure to remove excessacetic acid, the pH was slightly alkalized with saturated sodium bicarbonate solution, and then the residue was extracted and purified with ethyl acetate to obtain 4-((4-(1,4-dioxa-8-azaspiro[4.5]decan-8-yl)phenyl)thio)benzene-1,2-diamine (Chemical Formula **3-4,** 403 mg) as a yellow solid (yield 97%).
¹H NMR (CDCl₃, 400 MHz) δ 7.33(d, J=8.8Hz, 2H), 7.08(s, 1H), 6.93(m, 1H), 6.88(d, J=8.8Hz, 2H), 6.63(d, J=9.4Hz, 1H), 4.01(s, 4H), 3.35(m, 4H), 1.85(m, 4H)

### 4.3. Synthesis of methyl (5-((4-(1,4-dioxa-8-azaspiro[4.5]decan-8-yl)phenyl)thio)-1H-benzo[d]imidazol-2-yl)carbamate (Chemical Formula 1-4)

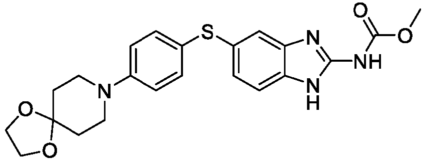

To a solution of 4-((4-(1,4-dioxa-8-azaspiro[4.5]decan-8-yl)phenyl)thio)benzene-1,2-diamine (Chemical Formula **3-4,** 350 mg, 0.98 mmol) in 5% acetic acid ethyl alcohol (7 mL), was added 1,3-bis(methoxycarbonyl)-S-methylisothiourea (509 mg, 2.47 mmol) and the mixture was reacted at 80°C for 24 hours. As the reaction time elapsed, a white precipitate was obtained from the reactants. Methyl alcohol (20 mL) was added at 40°C and filtered to obtain the product. The product was washed with methyl alcohol until the urea odor was removed, to obtain (5-((4-(1,4-dioxa-8-azaspiro[4.5]decan-8-yl)phenyl)thio)-1H-benzo[d]imidazol-2-yl)carbamate (Chemical Formula **1-4,** 210 mg) as a pure product (yield 49%).
¹H NMR (DMSO-d₆, 400 MHz) δ 11.80(m, 1H), 11.32(m, 1H), 7.33(m, 2H), 7.18(d, J = 8.4 Hz, 2H), 7.04(m, 1H), 6.93(d, J = 8.8 Hz, 2H), 3.90(s, 4H), 3.74(s, 3H), 3.28(m, 4H), 1.68(m, 4H)

### Preparation Example 5. Synthesis of Chemical Formula 1-5

The synthesis process of the thiobenzimidazole derivative of Chemical Formula 1-5 is shown by Reaction Scheme 5 presented below.

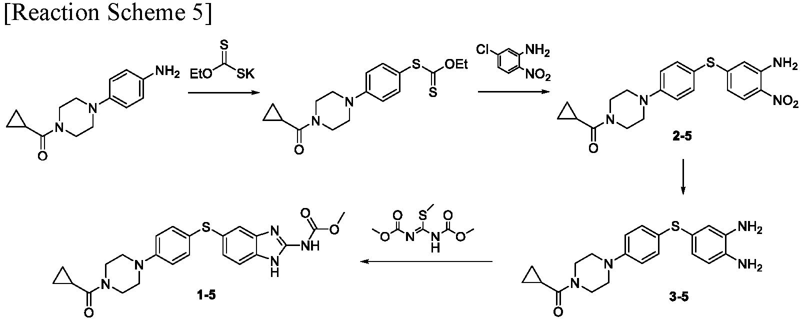

### 5.1. Synthesis of (1-(4-((3-amino-4-nitrophenyl)thio)phenyl)piperidin-4-yl)(cyclopropyl)methanone (Chemical Formula 2-5)

### (1) Synthesis of S-(4-(4-(cyclopropanecarbonyl)piperazin-1-yl)phenyl) O-ethyl carbonodithioate

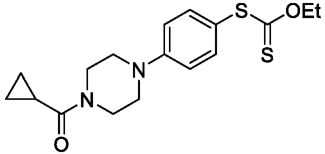

A mixed solution of (4-(4-aminophenyl)piperazin-1-yl)(cyclopropyl)methanone (1.22 g, 5.0 mmol) in distilled water (15 mL), sodium nitrite (525 mg) dissolved in distilled water (5 mL), and concentrated hydrochloric acid (0.995 mL) was prepared at 0°C in an ice bath. To the mixture prepared previously was slowly added dropwise a solution of Potassium ethylxanthate (1.552 g, 9.5 mmol) in distilled water (3 mL) at 50°C and the mixture was reacted for 1 hour. After the reaction was completed, the mixture was poured into water (800 mL) and extracted with EA (80 mL×2). The organic layer was dried over anhydrous magnesium sulfate, concentrated, and purified with ethyl acetate and n-hexane to obtain S-(4-(4-(cyclopropanecarbonyl)piperazin-1-yl)phenyl) O-ethyl carbonodithioate (450 mg) as a brown solid (yield 26%).
¹H NMR (CDCl₃, 400 MHz) δ 7.40(d, J=8.8Hz, 2H), 6.93(d, J=8.8Hz, 2H), 4.64(q, 2H), 3.87(m, 4H), 3.37(m, 4H), 1.77(m, 1H), 1.37(t, 3H), 1.06(m, 2H), 0.84(m, 2H)

### (2) Synthesis of (4-(4-((3-amino-4-nitrophenyl)thio)phenyl)piperazin-1-yl)(cyclopropyl)methanone (Chemical Formula 2-5)

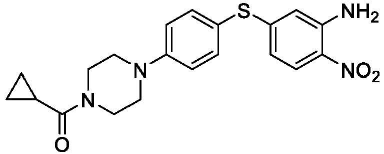

To a solution of S-(4-(4-(cyclopropanecarbonyl)piperazin-1-yl)phenyl)O-ethyl carbonodithioate (300 mg, 0.86 mmol) in THF/MeOH (10/15 mL) was added 5-chloro-2-nitroaniline (177 mg, 1.03 mmol), then sodium hydroxide powder (170 mg) dissolved in distilled water (4 mL) was added. The reaction mixture was reacted at 90°C for 5 hours, then the reaction mixture was separated and purified by a mixed solution of ethyl acetate and hexane (3:1, v/v) to obtain (4-(4-((3)-amino-4-nitrophenyl)thio)phenyl)piperazin-1-yl)(cyclopropyl)methanone (Chemical Formula **2-5,** 150 mg) as a yellow solid (yield 44%).
¹H NMR (CDCl₃, 400 MHz) δ 7.98(d, J=9.2Hz, 1H), 7.45(d, J=8.8Hz, 2H), 6.69(d, J=8.8Hz, 2H), 6.40(m, 1H), 6.29(s, 2H), 6.04(s, 2H), 3.89(m, 4H), 3.37(m, 4H), 1.78(m, 1H), 1.05(m, 2H), 0.84(m, 2H)

### 5.2. Synthesis of cyclopropyl(1-(4-((3,4-diaminophenyl)thio)phenyl)piperidin-4-yl)methanone (Chemical Formula 3-5)

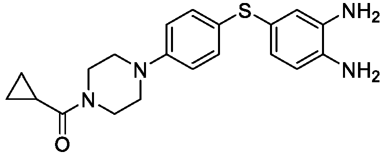

To a solution of (4-(4-((3-amino-4-nitrophenyl)thio)phenyl)piperazin-1-yl)(cyclopropyl)methanone (Chemical Formula **2-5,** 150 mg, 0.37 mmol) in acetic acid (3 mL), was added activated zinc powder (250 mg) and the mixture was reacted at room temperature for 12 hours. The reaction mixture was filtered, the filtrate was evaporated under reduced pressure to remove excess acetic acid, the pH was slightly alkalized with saturated sodium bicarbonate solution, and then the residue was extracted and purified with ethyl acetate to obtain cyclopropyl(4-(4-((3,4)-diaminophenyl)thio)phenyl)piperazin-1-yl)methanone (Chemical Formula **3-5,** 120 mg) as a yellow solid (yield 87%).
¹H NMR (CDCl₃, 400 MHz) δ 7.23(s, 1H), 7.04(d, J=8.8Hz, 2H), 6.58(d, J=8.8Hz, 2H), 6.43(d, J=9.4Hz, 2H), 4.94(br. s, 4H), 3.56(m, 4H), 3.35(m, 4H), 1.41(m, 1H), 0.78 (m, 2H), 0.53(m, 2H)

### 5.3. Synthesis of methyl (5-((4-(4-acetylpiperidin-1-yl)phenyl)thio)-1H-benzo[d]imidazol-2-yl)carbamate (Chemical Formula 1-5)

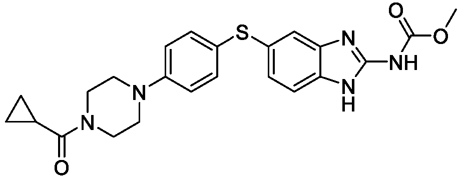

To a solution of cyclopropyl(4-(4-((3,4-diaminophenyl)thio)phenyl)piperazin-1-yl)methanone (Chemical Formula **3-5,** 70 mg, 0.19 mmol) in 5% acetic acid ethyl alcohol (1.5 mL) was added 1,3-bis(methoxycarbonyl)-S-methylisothiourea (110 mg, 0.53 mmol), then the mixture was heated to reflux and reacted for 5 hours. Methyl alcohol (7 mL) was added to the reactants at 40°C. The reaction mixture was filtered, and the filtrate was washed with additional methyl alcohol until the urea odor was removed to obtain methyl (5-((4-(4-acetylpiperidin-1-yl))phenyl)thio)-1H-benzo[d]imidazol-2-yl)carbamate (Chemical Formula **1-5, 70** mg) as a pure product (yield 81.6%).
¹H NMR (DMSO-d6, 400 MHz) δ 11.86(m, 1H), 11.43(m, 1H), 7.33(m, 2H), 7.23(d, J=8.8Hz, 2H), 7.05(m, 1H), 6.94(d, J=8.8Hz, 2H), 3.80(s, 2H), 3.74(s, 3H), 3.59(s, 2H), 3.17(m, 4H), 2.02(m, 1H), 0.75(m, 4H)

### Example 6. Synthesis of Chemical Formula 1-6

The synthesis process of the thiobenzimidazole derivative of Chemical Formula 1-6 is shown by Reaction Scheme 6 presented below.

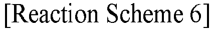

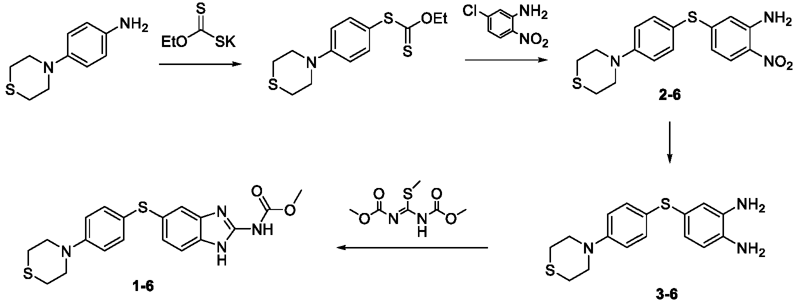

### 6.1. Synthesis of 2-nitro-5-((4-thiomorpholinophenyl)thio)aniline (Chemical Formula 2-6)

### (1) Synthesis of O-ethyl S-(4-thiomorpholinophenyl) carbonodithioate

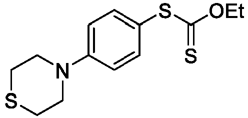

A mixed solution of 4-(4-Thiomorpholinyl)benzenamine (1.56 g, 8.0 mmol) in distilled water (20 mL), sodium nitrite (672 mg) dissolved in distilled water (5 mL), and concentrated hydrochloric acid (1.2 mL) was prepared at 0°C in an ice bath. To the mixture prepared previously was slowly added dropwise a solution of Potassium ethylxanthate (1.94 g, 12.1 mmol) in distilled water (3 mL) at 50°C and the mixture was reacted for 1 hour. After the reaction was completed, the mixture was poured into water (100 mL) and extracted with EA (100 mL×2). The organic layer was dried over anhydrous magnesium sulfate, concentrated, and the residue was purified with ethyl acetate and n-hexane to obtain O-ethyl S-(4-thiomorpholinophenyl)carbonodithioate (Chemical Formula **3-7,** 600 mg) as a brown solid (yield 75%).
¹H NMR (CDCl₃, 400 MHz) δ 7.38(d, J=8.4Hz, 2H), 6.90(d, J=8.8Hz, 2H), 4.65(q, 2H), 3.71(m, 4H), 2.76(m, 4H), 1.37(t, 3H)

### (2) Synthesis of 2-nitro-5-((4-thiomorpholinophenyl)thio)aniline (Chemical Formula 2-6)

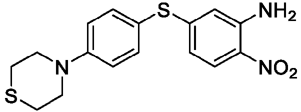

To a solution of O-ethyl S-(4-thiomorpholinophenyl)carbonodithioate (600 mg, 2.0 mmol) in THF/MeOH (20/30 mL) was added 5-chloro-2-nitroaniline (346 mg, 2.1 mmol), then sodium hydroxide powder (336 mg) dissolved in distilled water (7 mL) was added. The reaction mixture was reacted at 90°C for 5 hours, then separated and the residue was purified by a mixed solution of ethyl acetate and hexane to obtain 2-nitro-5-((4-thiomorpholinophenyl)thio)aniline (500 mg) as a yellow solid (yield 72%).
¹H NMR (CDCl₃, 400 MHz) δ 7.99(d, J=8.8Hz, 1H), 7.42(d, J=8.8Hz, 2H), 6.93(d, J=8.4Hz, 2H), 6.42(m, 1H), 6.28(s, 1H), 6.03(s, 2H), 3.72(m, 4H), 2.77(m, 4H)

### 6.2. Synthesis of 4-((4-thiomorpholinophenyl)thio)benzene-1,2-diamine (Chemical Formula 3-6)

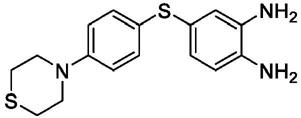

To a solution of 2-nitro-5-((4-thiomorpholinophenyl)thio)aniline (500 mg, 1.44 mmol) in acetic acid (8 mL), was added activated zinc powder (500 mg) and the mixture was reacted at room temperature for 5 hours. The reaction mixture was filtered, the filtrate was evaporated under reduced pressure to remove excess acetic acid, the pH was slightly alkalized with sodium bicarbonate, and then the filtrate was extracted and the residue was purified with ethyl acetate to obtain 4-((4-thiomorpholinophenyl)thio)benzene-1,2-diamine (398 mg) as a yellow solid (yield 87%).
¹H NMR (CDCl₃, 400 MHz) δ 7.83(d, J=8.8Hz, 2H), 7.40(d, J=8.8Hz, 2H), 7.20 and 6.93(2d, J=8.4Hz, 2H), 6.28(s, 1H), 4.95(br, s, 4H), 3.72(m, 4H), 2.67(m, 4H)

### 6.3. Synthesis of methyl (5-((4-thiomorpholinophenyl)thio)-1H-benzo[d]imidazol-2-yl)carbamate (Chemical Formula 1-6)

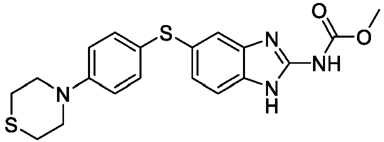

To a solution of 4-((4-thiomorpholinophenyl)thio)benzene-1,2-diamine (158 mg, 0.5 mmol) in 5% acetic acid ethyl alcohol (3 mL), was added 1,3-bis(methoxycarbonyl)-S-methylisothiourea (260 mg, 1.26 mmol) and the mixture was reacted at 80°C for 24 hours. As the reaction time elapsed, a white precipitate was obtained from the reactants. Methyl alcohol (20 mL) was added at 40°C and the mixture was filtered to obtain the product. The product was further washed with methyl alcohol until the urea odor was removed, to obtain methyl (5-((4-thiomorpholinophenyl)thio)-1H-benzo[d]imidazol-2-yl)carbamate (120 mg) as a pure product (yield 60.3%).
¹H NMR (DMSO-d₆, 400 MHz) δ 11.83(m, 1H), 11.38(m, 1H), 7.33(m, 2H), 7.22(d, J=8.4Hz, 2H), 7.04(d, J=8.0Hz, 1H), 6.92(d, J=Hz, 2H), 3.74(s, 3H), 3.56(m, 4H), 2.63(m, 4H)

### Preparation Example 7. Synthesis of Chemical Formula 1-7

The synthesis process of the thiobenzimidazole derivative of Chemical Formula 1-7 is shown by Reaction Scheme 7 presented below.

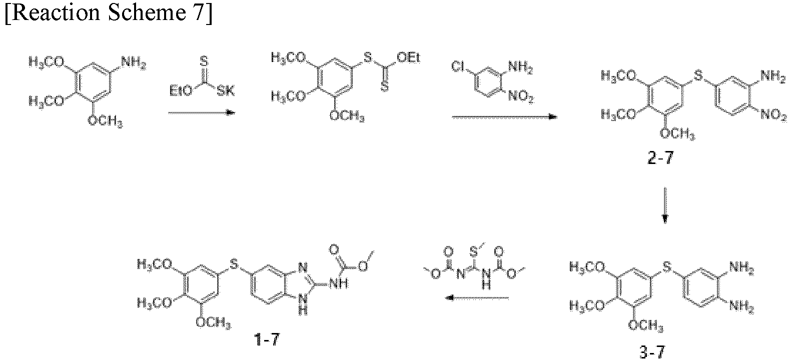

### 7.1. Synthesis of 2-nitro-5-((3,4,5-trimethoxyphenyl)thio)aniline (Chemical Formula 2-7)

### (1) Synthesis of O-ethyl S-(3,4,5-trimethoxyphenyl) carbonodithioate

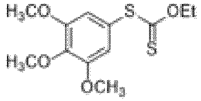

To a solution of 3,4,5-trimethoxyaniline (1.48 g, 8.07 mmol) in distilled water (22 mL) was added a mixed solution of sodium nitrite (1.2 eq, 0.672 g) in distilled water (5 mL) and concentrated hydrochloric acid (1.2 mL), to which was added a solution of potassium O-ethyl carbonodithioate (1.95 g, 1.5 eq) in distilled water (3 mL) and the reaction mixture was stirred for 2 hours. After the reaction was completed, the solvent was removed by evaporation under reduced pressure and the residue was separated by ethyl acetate and n-hexane (1:9, v/v). A second separation was performed by ethyl acetate and n-hexane (1:3, v/v) to obtain O-ethyl S-(3,4,5-trimethoxyphenyl)carbonodithioate (0.9 g) as a light yellow solid (yield 39%).
¹H NMR (CDC1₃, 400 MHz) δ 6.76(s, 2H), 4.66(q, 2H), 3.91(s, 3H), 3.88(s, 6H), 1.37(t, 3H)

### (2) Synthesis of 2-nitro-5-((3,4,5-trimethoxyphenyl)thio)aniline (Chemical Formula 2-7)

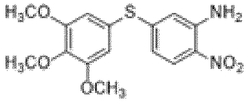

To a solution of O-ethyl S-(3,4,5-trimethoxyphenyl) carbonodithioate (0.68 g, 2.36 mmol) in THF (24 mL) was added 5-chloro-2-nitroaniline (0.416 g, 2.41 mmol) in methanol (40 mL), to which was added sodium hydroxide (0.384 g, 9.60 mmol)) dissolved in distilled water (8 mL). The reaction mixture was maintained at 70°C and reacted for 20 hours. The reaction mixture was cooled to room temperature, the pH was adjusted to pH 8, then the combined solvent was removed by evaporation under reduced pressure and the residue was partitioned between ethyl acetate and n-hexane (1:3, v/v) to afford 2-nitro-5-((3,4,5-trimethoxyphenyl)thio)aniline (Chemical Formula **2-7,** 0.35 g) as a yellow solid crystal (yield 44%).
¹H NMR (CDC1₃, 400 MHz) δ 8.02(d, J=8.8Hz, 1H), 6.80(s, 2H), 6.46(d, J=8.8Hz, 1H), 6.35(s, 1H), 6.08(s, 2H), 3.93(s, 3H), 3.88(s, 6H)

### 7.2. Synthesis of 4-((3,4,5-trimethoxyphenyl)thio)benzene-1,2-diamine (Chemical Formula 3-7)

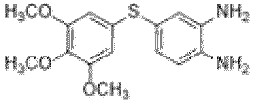

To a solution of 2-nitro-5-((3,4,5-trimethoxyphenyl)thio)aniline (0.300 g, 0.89 mmol) in ethyl alcohol (10 mL) was added tin(II) chloride (1.2 g), and the reaction mixture was heated to reflux for 20 hours. The solvent in the reaction mixture was removed by evaporation under reduced pressure, the pH was adjusted to pH 8 or higher with 2N-NaOH, and then the residue was extracted with ethyl acetate. Extraction was difficult due to excess compound, and thus it was done after filtration. The resulting mixture was dry filtered and the solvent was removed by evaporation under reduced pressure to afford 4-((3,4,5-trimethoxyphenyl)thio)benzene-1,2-diamine (Chemical Formula **3-7,** 0.220 g) as a brown product (yield 80.6%).
¹H NMR (CDC1₃, 400 MHz) δ 6.89(m, 2H), 6.71(d, J=8.0Hz, 1H), 6.48(s, 2H), 3.83(s, 3H), 3.78(s, 6H)3.51(s, 2H), 3.40(s, 2H)

### 7.3. Synthesis of methyl (5-((3,4,5-trimethoxyphenyl)thio)-1H-benzo[d]imidazol-2-yl)carbamate (Chemical Formula 1-7)

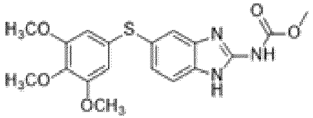

To a solution of 4-((3,4,5-trimethoxyphenyl)thio)benzene-1,2-diamine (0.1 g, 0.326 mmol, 1.0 eq) in 5% acetic acid in EtOH (5 mL) was added 1,3-bis(methoxycarbonyl)-S-methylisothiourea (75 mg, 0.485 mmol, 1.1 eq), the mixture was warmed and stirred at 80°C for 3 hours. The reaction mixture was monitored by TLC to confirm when the reaction was completed and cooled. MeOH (10 mL) was added, and the reaction mixture was filtered. The residue was washed with plenty of MeOH to remove urea or the reaction product was purified by column chromatography to obtain methyl (5-(furan-2-ylthio)-1H-benzo[d]imidazol-2-yl)carbamate (Chemical Formula **1-7,** 0.105 g) as a white solid (yield 82.6%).
¹H NMR (400 MHz, DMSO-d₆): δ 11.94(m, 1H), 11.43(m, 1H), 7.48(s, 1H), 7.43(d, J=6.8hz, 1H), 7.18(d, J=8.4Hz, 1H), 6.53(s, 2H), 3.75(s, 3H), 3.66(s, 6H), 3.62(s, 3H)

### Preparation Example 8. Synthesis of Chemical Formula 1-8

The synthesis process of the thiobenzimidazole derivative of Chemical Formula 1-8 is shown by Reaction Scheme 8 presented below.

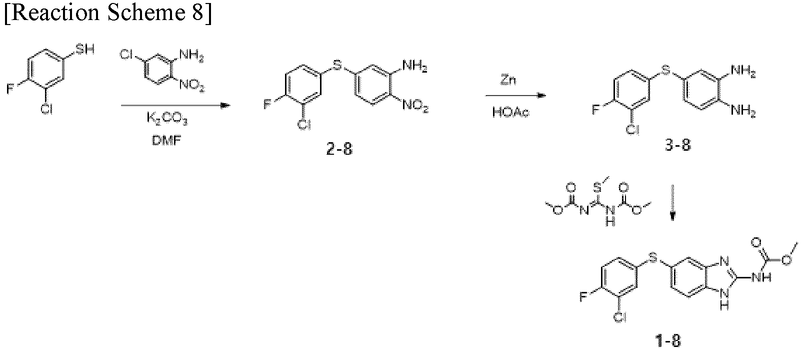

### 8.1. Synthesis of 5-((3-chloro-4-fluorophenyl)thio)-2-nitroaniline (Chemical Formula 2-8)

A solution of 3-chloro-4-fluorobenzenethiol (1.41 g, 8.69 mmol) in DMF (13 mL) was reacted with a mixed solution of potassium carbonate (1.24 g, 9.01 mmol) and 5-chloro-2-nitroaniline (1.5 g, 8.69 mmol) at 65°C for 20 hours. The reaction mixture was cooled to room temperature and then extracted with organic solvent while the pH was maintained at 7 to 8. The resulting reaction mixture was separated by column chromatography with methylene chloride and methanol (9:1. v/v) solvent to afford 5-((3-chloro-4-fluorophenyl)thio)-2-nitroaniline (Chemical Formula **2-8,** 1.94 g) as a light brown solid with an R_{f} value of 0.20 (yield 75%).
¹H NMR (CDC1₃, 400 MHz) δ 8.04(d, J=8.8Hz, 1H), 7.63(d, J=6.8Hz, 1H), 7.45(m, 1H), 7.26(m, 1H), 6.44(m, 2H), 6.08(s, 2H)

### 8.2. Synthesis of 4-((3-chloro-4-fluorophenyl)thio)benzene-1,2-diamine (Chemical Formula 3-8)

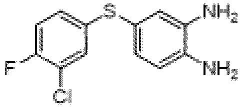

5-((3-chloro-4-fluorophenyl)thio)-2-nitroaniline (Chemical Formula **2-8,** 1.0 g, 3.35 mmol) was dissolved in acetic acid (10 mL). To the solution was added Zinc power (1. 58 g), and the reaction mixture was stirred at room temperature for 10 hours to obtain 4-((3-chloro-4-fluorophenyl)thio)benzene-1,2-diamine (Chemical Formula **3-8,** 0.90 g) as a light brown solid (yield 80%).
¹H NMR (CDC1₃, 400 MHz) δ 7.18(d, J=6.8Hz, 1H), 7.03(m, 2H), 6.87(m, 2H), 6.73(d, J=8.0Hz, 2H), 3.57(s, 2H), 3.42(s, 2H)

### 8.3. Synthesis of methyl (5-((3-chloro-4-fluorophenyl)thio)-1H-benzo[d]imidazol-2-yl)carbamate (Chemical Formula 1-8)

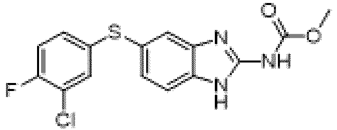

To a solution of 4-((3-chloro-4-fluorophenyl)thio)benzene-1,2-diamine (Chemical Formula **3-8,** 0.270 g, 0.969 mmol, 1.0 eq) in 5% acetic acid in EtOH (7 mL) was added 1,3-bis(methoxycarbonyl)-S-methylisothiourea (517 mg), and the mixture was warmed and stirred at 80°C for 5 hours. The reaction mixture was monitored by TLC to confirm when the reaction was completed, then cooled. MeOH (10 mL) was added. The mixture was filtered. The residue was washed with plenty of MeOH to remove urea to obtain methyl (5-((3-chloro-4-fluorophenyl)thio)-1H-benzo[d]imidazol-2-yl)carbamate (Chemical Formula **1-8,** 0.263 g) as a white solid (yield 75%).

¹H NMR (400 MHz, DMSO-d₆): δ 12.01(m, 1H), 11.50(m, 1H), 7.55(s, 1H), 7.47(m, 1H), 7.38-7.27(m, 2H), 7.23(d, J=8.0Hz, 1H), 7.11(m, 1H), 3.76(s, 3H)

### Preparation Example 9. Synthesis of Chemical Formula 1-9

The synthesis process of the thiobenzimidazole derivative of Chemical Formula 1-9 is shown by Reaction Scheme 9 presented below.

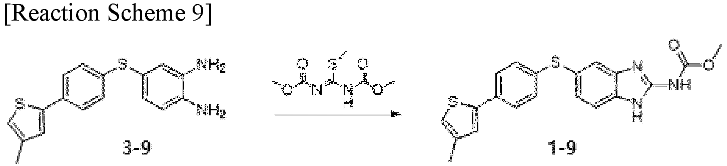

### 9.1. Synthesis of 4-((4-(4-methylthiophen-2-yl)phenyl)thio)benzene-1,2-diamine (Chemical Formula 1-9)

4-((3-chloro-4-fluorophenyl)thio)benzene-1,2-diamine (Chemical Formula **3-9**) was prepared in the same manner as in Preparation Example 1, except (4-methylthiophen-2-yl)boronic acid was used instead of (5-methylthiophen-2-yl)boronic acid as an intermediate reactant. To a solution of 4-((3-chloro-4-fluorophenyl)thio)benzene-1,2-diamine (Chemical Formula **3-9,** 0.080 g, 0.256 mmol) in 5% acetic acid in EtOH (4 mL) was added 1,3-bis(methoxycarbonyl)-S-methylisothiourea (190 mg), and the mixture was warmed and stirred at 80°C for 3 hours. The reaction mixture was monitored by TLC to confirm when the reaction was completed, then cooled. MeOH (5 mL) was added. The reaction mixture was filtered. The residue was washed with plenty of MeOH to remove urea to obtain methyl (5-((4-(4-methylthiophen-2-yl)phenyl)thio)-1H-benzo[d]imidazol-2-yl)carbamate (Chemical Formula **1-9,** 0.07 g) as a white solid (yield 70%).
¹H NMR (400 MHz, DMSO-d₆): δ 2.21(s, 3H), 3.76(s, 3H), 7.02-7.28(m, 5H), 7.45(d, J=8.8Hz, 2H), 7.53(m, 2H), 11.42(m, 1H), 12.02(m, 1H)

### Preparation Example 10. Synthesis of Chemical Formula 1-10

The synthesis process of the thiobenzimidazole derivative of Chemical Formula 1-10 is shown by Reaction Scheme 10 presented below.

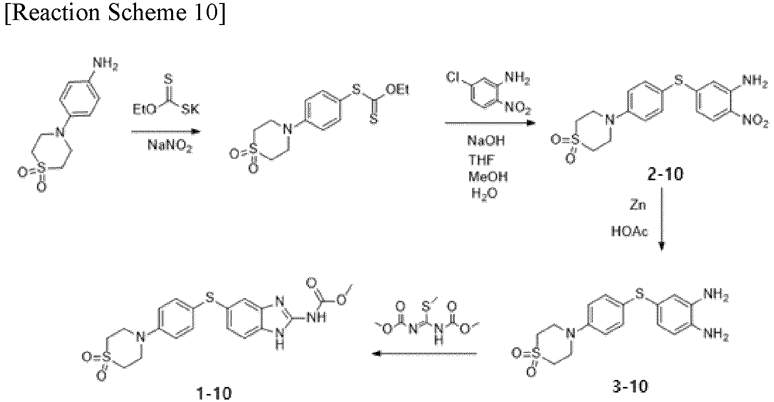

### 10.1. Synthesis of 4-(4-((3-amino-4-nitrophenyl)thio)phenyl)thiomorpholine 1,1-dioxide (Chemical Formula 2-10)

### (1) Synthesis of S-(4-(1,1-dioxidothiomorpholino)phenyl)O-ethyl carbonodithioate

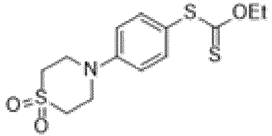

To a solution of 4-(4-aminophenyl)thiomorpholine 1,1-dioxide (0.727 g, 3.21 mmol) in water (8.4 mL) was added potassium O-ethyl carbonodithioate (0.780 g) dissolved in water (1.2 mL), to which was added a solution of NaNO₂ (0.264 g, 3.80 mmol) in distilled water (2.4 mL), and then the reaction mixture was stirred at 80°C for 16 hours. After the reaction was completed, the solvent was removed by evaporation under reduced pressure. The residue was extracted with ethyl acetate, then the solvent was removed and the residue was partitioned between ethyl acetate and methanol (9:1, v/v) to afford S-(4-(1,1-dioxidothiomorpholino)phenyl)O-ethyl carbonodithioate (0.981 g) as a yellow solid (yield 89.2%).
¹H NMR (CDC1₃, 400 MHz) δ 7.44(d, J=8.8Hz, 2H), 6.96(d, J=8.8Hz, 2H), 4.66(q, 2H), 3.98(m, 4H), 3.15(m, 4H), 1.38(t, 3H)

### (2) Synthesis of 4-(4-((3-amino-4-nitrophenyl)thio)phenyl)thiomorpholine 1,1-dioxide (Chemical Formula 2-10)

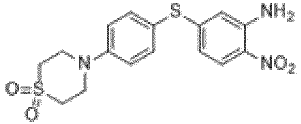

To a solution of S-(4-(1,1-dioxidothiomorpholino)phenyl) O-ethyl carbonodithioate (0.950 g, 2.866 mmol) in THF (30 mL) was added a solution of 5-chloro-2-nitroaniline (0.500 g, 1.738 mmol) in MeOH (50 mL), to which was added NaOH (0.550 g) dissolved in distilled water (10 mL). The reaction mixture was reacted at 80°C for 16 hours. After the reaction was completed, the pH was adjusted to 8, and the solvent was removed by evaporation under reduced pressure. The residue was extracted with ethyl acetate. The residue was again partitioned between ethyl acetate and methanol (9: 1, v/v), and then recrystallized to afford 4-(4-((3-amino-4-nitrophenyl)thio)phenyl)thiomorpholine 1,1-dioxide (Chemical Formula **2-10,** 0.850 g) as a white solid (yield 78.1%).
¹H NMR (CDC1₃, 400 MHz) δ 8.00(d, J=9.2Hz, 1H), 7.50(d, J=8.8Hz, 2H), 6.98)d, J=8.8Hz, 2H), 6.41(m, 1H), 6.33(s, 1H), 6.05(s, 2H), 3.99(m, 4H), 3.17(m, 4H)

### 10.2. Synthesis of 4-(4-((3,4-diaminophenyl)thio)phenyl)thiomorpholine 1,1-dioxide (Chemical Formula 3-10)

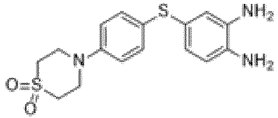

To a solution of 4-(4-((3-amino-4-nitrophenyl)thio)phenyl)thiomorpholine 1,1-dioxide (Chemical Formula **2-10,** 0.800 g, 2.108 mmol) in HOAc (8.0 mL) was added Zinc (0.500 mg) and the mixture was reacted at 50°C for 12 hours. After the reaction was completed, the solvent was removed by evaporation under reduced pressure, and triturated with distilled water. The pH was adjusted to 8 with an aqueous sodium hydroxide solution. The resulting product was extracted four times with ethyl acetate, the solvent was removed and the residue was partitioned between ethyl acetate and methanol (9:1, v/v/), and then was recrystallized to afford 4-(4-((3,4-diaminophenyl)thio)phenyl)thiomorpholine 1,1-dioxide (Chemical Formula **3-10,** 0.210 g) as a white solid (yield 81.4%).
¹H NMR (CDC1₃, 400 MHz) δ 7.24(d, J=9.2Hz, 2H), 6.82(m, 4H), 6.69(d, J=8.0Hz, 1H), 3.84(m, 4H), 3.47(m, 4H), 3.12(m, 4H)

### 10.3. Synthesis of methyl (5-((4-(1,1-dioxidothiomorpholino)phenyl)thio)-1H-benzo[d]imidazol-2-yl)carbamate (Chemical Formula 1-10)

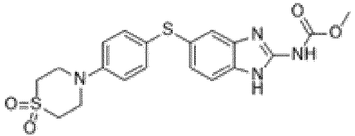

To a solution of 4-(4-((3,4-diaminophenyl)thio)phenyl)thiomorpholine 1,1-dioxide (Chemical Formula **3-10,** 0.180 g, 0.516 mmol) in 5% acetic acid in EtOH (3 mL) was added 1,3-bis(methoxycarbonyl)-S-methylisothiourea (267 mg, 1.29 mmol) and the mixture was warmed and stirred at 80°C for 3 hours. The reaction mixture was monitored by TLC to confirm when the reaction was completed, then cooled. MeOH (5 mL) was added. Then, the resulting mixture was filtered. The residue was washed with plenty of MeOH to remove urea to obtain methyl (5-((4-(1,1-dioxidothiomorpholino)phenyl)thio)-1H-benzo[d]imidazol-2-yl)carbamate (Chemical Formula **1-10,** 0.150 g) as a white solid (yield 67%).
¹H NMR (400 MHz, DMSO-d₆): δ 11.80(m, 1H), 11.35(m, 1H), 7.35(s, 2H), 7.22(d, J=8.8Hz, 2H), 7.07(m, 1H), 7.02(d, J=8.8Hz, 2H), 3.78(m, 4H), 3.74(, 3H), 3.10(m, 4H)

### Preparation Example 11. Synthesis of Chemical Formula 1-11

The synthesis process of the thiobenzimidazole derivative of Chemical Formula 1-11 is shown by Reaction Scheme 11 presented below.

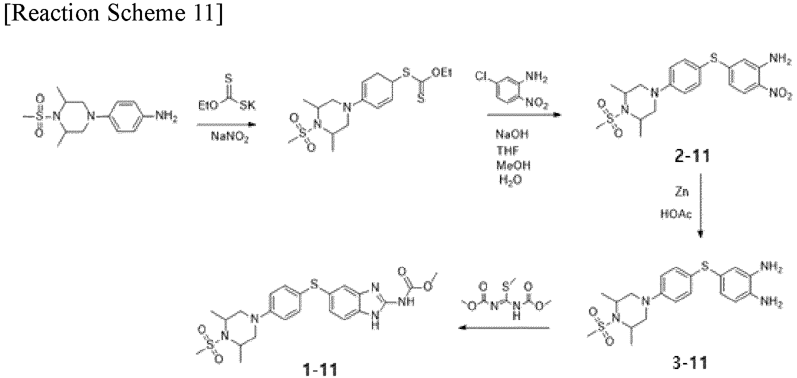

### 11.1. Synthesis of 5-((4-(3,5-dimethyl-4-(methylsulfonyl)piperazin-1-yl)phenyl)thio)-2-nitroaniline (Chemical Formula 2-11)

### (1) Synthesis of S-(4-(3,5-dimethyl-4-(methylsulfonyl)piperazin-1-yl)cyclohexa-2,4-dien-1-yl)O-ethyl carbonodithioate

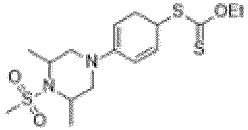

A solution of 4-(3,5-dimethyl-4-(methylsulfonyl)piperazin-1-yl)aniline (2.0 g, 7.05 mmol) in hydrochloric acid (1.2 mL) and water (23 mL) was cooled in a brine ice bath. To the solution was slowly added dropwise a solution of NaNO₂ (0.650 g) in distilled water (5 mL) under the same conditions for 30 minutes. The reaction mixture was adjusted to room temperature. A solution of potassium O-ethyl carbonodithioate (1.8 g) in water (5 mL) was added at 40°C. The mixture was reacted for 4 hours. After the reaction was completed, the solvent was removed by evaporation under reduced pressure. The resulting product was extracted with ethyl acetate and the solvent was removed. Then the residue was separated by silica gel chromatography using ethyl acetate and hexane (1:3, v/v) to obtain S-(4-(3,5-dimethyl-4-(methylsulfonyl)piperazin-1-yl)cyclohexa-2,4-dien-1-yl)O-ethyl carbonodithioate (1.5 g) as a yellow solid (yield 54.5%).
¹H NMR (CDC1₃, 400 MHz) δ 7.40(d, J=8.8Hz, 2H), 6.93(d, J=9.2Hz, 2H), 4.65(q, 2H), 4.22(m, 2H), 3.56(d, J=12.4Hz, 2H), 3.11(m, 2H), 2.93(s, 3H), 1.53(d, J=6.8Hz, 6H), 1.40(t, 3H)

### (2) Synthesis of 5-((4-(3,5-dimethyl-4-(methylsulfonyl)piperazin-1-yl)phenyl)thio)-2-nitroaniline (Chemical Formula 2-11)

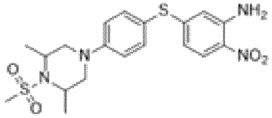

To a solution of S-(4-(3,5-dimethyl-4-(methylsulfonyl)piperazin-1-yl)cyclohexa-2,4-dien-1-yl)O-ethyl carbonodithioate (1.2 g, 3.07 mmol) in THF (30 mL) was added a solution of 5-chloro-2-nitroaniline (0.520 g, 3.01 mmol) in MeOH (50 mL), to which was added a solution of NaOH (0.480 g, 12 mmol) dissolved in distilled water (10 mL). The reaction mixture was reacted at 90°Cfor 7 hours. After the reaction was completed, the pH was adjusted to 8, and the solvent was removed by evaporation under reduced pressure. The resulting product was extracted with ethyl acetate and washed with water. The residue was again partitioned between ethyl acetate and methanol (1:3, v/v) and then recrystallized to afford 5-((4-(3,5-dimethyl-4-(methylsulfonyl)piperazin-1-yl)phenyl)thio)-2-nitroaniline (Chemical Formula **2-11,** 0.950 g) as a white solid (yield 71%).
¹H NMR (CDC1₃, 400 MHz) δ 7.98(d, J=8.8Hz, 1H), 7.46(d, J=8.8Hz, 2H), 6.96(d, J=8.8Hz, 2H), 6.40(d, J=9.2Hz, 1H), 6.32(s, 1H), 6.05(s, 2H), 4.25(m, 2H), 3.56(d, J=12.4Hz, 2H), 3.12(m, 2H), 2.95(s, 3H), 1.55(d, J=6.8Hz, 6H)

### 11.2. Synthesis of 4-(4-((3,4-diaminophenyl)thio)phenyl)thiomorpholine 1,1-dioxide (Chemical Formula 3-11)

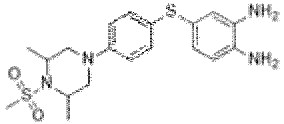

To a solution of 5-((4-(3,5-dimethyl-4-(methylsulfonyl)piperazin-1-yl)phenyl)thio)-2-nitroaniline (Chemical Formula **2-11,** 0.170 g, 0.389 mmol) in HOAc (2.0 mL) was added Zinc (0.150 mg) and the mixture was reacted at 50°C for 15 hours. After the reaction was completed, the solvent was removed by evaporation under reduced pressure, and triturated with distilled water. The pH was adjusted to 8 with an aqueous sodium hydroxide solution. The resulting product was extracted four times with ethyl acetate. Then, the solvent was removed and the residue was partitioned between ethyl acetate and methanol (9:1, v/v/), and then was recrystallized to afford 4-(4-((3,4-diaminophenyl)thio)phenyl)thiomorpholine 1,1-dioxide (Chemical Formula **3-11,** 0.110 g) as a white solid (yield 70.2%).
¹H NMR (CDC1₃, 400 MHz) δ 7.25(d, J=8.8Hz, 2H), 6.81(m, 4H), 6.67(d, 8.0Hz, 1H), 4,15(m, 2H), 3.43(m, 2H), 3.37(d, J=12.0Hz, 4H), 3.29(m, 2H), 2.92(s, 3H), 1.30(d, J=6.8Hz, 6H)

### 11.3. Synthesis of methyl (5-((4-(3,5-dimethyl-4-(methylsulfonyl)piperazin-1-yl)phenyl)thio)-1H-benzo[d]imidazol-2-yl)carbamate (Chemical Formula 1-11)

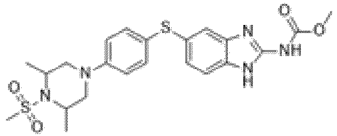

To a solution of 4-((4-(3,5-dimethyl-4-(methylsulfonyl)piperazin-1-yl)phenyl)thio)benzene-1,2-diamine (Chemical Formula **3-11,** 0.080 g, 0.197 mmol) in 5% acetic acid in EtOH (1.5 mL) was added 1,3-bis(methoxycarbonyl)-S-methylisothiourea (102 mg, 4.93 mmol) and the mixture was warmed and stirred at 80°C for 3 hours. The reaction mixture was monitored by TLC to confirm when the reaction was completed, then cooled. MeOH (5 mL) was added. The mixture was filtered. The residue was washed with plenty of MeOH to remove urea to obtain methyl (5-((4-(3,5-dimethyl-4-(methylsulfonyl)piperazin-1-yl)phenyl)thio)-1H-benzo[d]imidazol-2-yl)carbamate (Chemical Formula **1-11,** 0.08 g) as a white solid (yield 63%).
¹H NMR (400 MHz, DMSO-d₆): δ 11.82(m, 1H), 11.31(m, 1H), 7.33(m, 2H), 7.22(d, J=8.8Hz, 2H), 7.04(m, 1H), 6.94(d, J=8.8Hz, 2H), 4.03(m, 2H), 3.74(s, 3H), 3.59(m, 2H), 2.96(s, 3H), 2.90(m, 2H), 1.33(d, J=6.8Hz, 6H)

### Preparation Example 12. Synthesis of Chemical Formula 1-12

The synthesis process of the thiobenzimidazole derivative of Chemical Formula 1-12 is shown by Reaction Scheme 12 presented below.

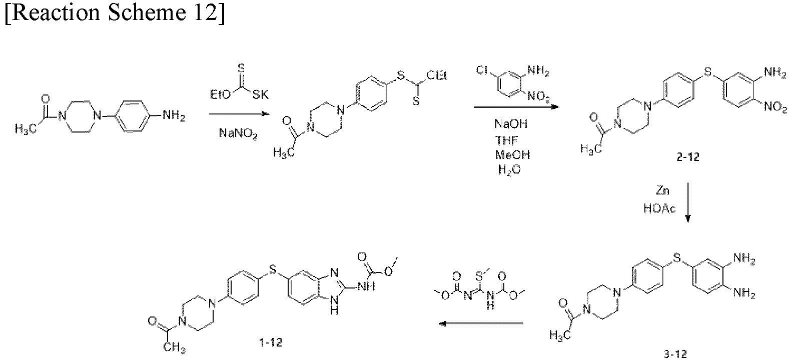

### 12.1. Synthesis of 1-(4-(4-((3-amino-4-nitrophenyl)thio)phenyl)piperazin-1-yl)ethan-1-one (Chemical Formula 2-12)

### (1) Synthesis of S-(4-(4-acetylpiperazin-1-yl)phenyl) O-ethyl carbonodithioate

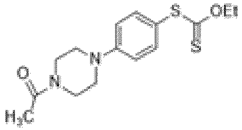

A solution of 1-(4-(4-aminophenyl)piperazin-1-yl)ethan-1-one (0.620 g, 2.83 mmol) in hydrochloric acid (0.4 mL) and water (7 mL) was cooled in a brine ice bath. To the solution was slowly added dropwise a solution of NaNO₂ (0.236 g) in distilled water (1.7 mL) under the same conditions for 20 minutes. The reaction mixture was allowed to warm to room temperature. A solution of potassium O-ethyl carbonodithioate (0.64 g) in water (2 mL) was added at 50°C. The mixture was reacted for 30 minutes. After the reaction was completed, the solvent was removed by evaporation under reduced pressure. The resulting product was extracted with ethyl acetate, the solvent was removed, and then the residue was separated by silica gel chromatography using ethyl acetate and hexane (3: 1, v/v) to obtain S-(4-(4-acetylpiperazin-1-yl)phenyl) O-ethyl carbonodithioate (0.630 g) as a yellow solid (yield 68.7%).
¹H NMR (CDC1₃, 400 MHz) δ 7.40(d, J=8.8Hz, 2H), 6.94(d, J=8.4Hz, 2H), 4.64(q, 2H), 3.80(m, 2H), 3.67(m, 2H), 3.33(m, 4H), 2.17(s, 3H), 1.38(t, 3H)

### (2) Synthesis of 1-(4-(4-((3-amino-4-nitrophenyl)thio)phenyl)piperazin-1-yl)ethan-1-one (Chemical Formula 2-12)

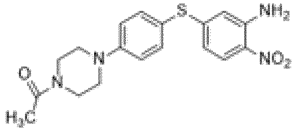

To a solution of S-(4-(4-acetylpiperazin-1-yl)phenyl) O-ethyl carbonodithioate (0.630 g, 1.94 mmol) in THF (20 mL) was added a solution of 5-chloro-2-nitroaniline (0.338 g, 1.95 mmol) in MeOH (42 mL), to which was added NaOH (0.312 g, 12 mmol) dissolved in water (6.5 mL). The reaction mixture was reacted at 90°C for 10 hours. After the reaction was completed, the pH was adjusted to 8, and then the solvent was removed by evaporation under reduced pressure. The resulting product was extracted with ethyl acetate and washed with water. The crude product was again partitioned between ethyl acetate and methanol (1:3, v/v) and then was recrystallized to afford 1-(4-(4-((3-amino-4-nitrophenyl)thio)phenyl)piperazin-1-yl)ethan-1-one (Chemical Formula **2-12,** 0.510 g) as a white solid (yield 70%).
¹H NMR (CDC1₃, 400 MHz) δ 7.86(d, J=9.2Hz, 1H), 7.44(s, 2H), 7.41(d, J=8.4Hz, 2H), 7.08(d, J=8.4Hz, 2H), 6.53(s, 1H), 6.27(d, J=9.2Hz, 1H), 3.60(m, 4H), 3.32(m, 2H), 3.25(m, 2H), 2.05(s, 3H)

### 12.2. Synthesis of 1-(4-(4-((3,4-diaminophenyl)thio)phenyl)piperazin-1-yl)ethan-1-one (Chemical Formula 3-12)

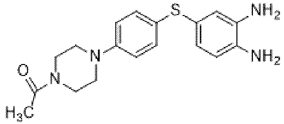

To a solution of 1-(4-(4-((3-amino-4-nitrophenyl)thio)phenyl)piperazin-1-yl)ethan-1-one (Chemical Formula **2-12,** 0.180 g, 0.483 mmol) in HOAc (2.0 mL) was added Zinc (0.150 mg) and the mixture was reacted at 50°C for 10 hours. After the reaction was completed, the solvent was removed by evaporation under reduced pressure, triturated with distilled water, and the pH was adjusted to 8 with an aqueous sodium hydroxide solution. The resulting product was extracted four times with ethyl acetate, the solvent was removed and the residue was partitioned between ethyl acetate and methanol (9:1, v/v/), and then was recrystallized to afford 1-(4-(4-((3,4-diaminophenyl)thio)phenyl)piperazin-1-yl)ethan-1-one (Chemical Formula **3-12,** 0.140 g) as a white solid (yield 85%).
¹H NMR (CDC1₃, 400 MHz) δ 7.25(d, J=8.8Hz, 2H), 6.85(d, J=8.4Hz, 2H), 6.77(m, 2H), 6.67(d, J=7.6Hz, 1H), 3.79(m, 2H), 3.62(m, 2H), 3.43(m, 4H), 3.18(m, 4H), 2.15(s, 3H)

### 12.3. Synthesis of methyl (5-((4-(4-acetylpiperazin-1-yl)phenyl)thio)-1H-benzo[d]imidazol-2-yl)carbamate (Chemical Formula 1-12)

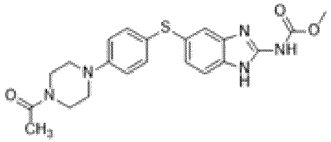

To a solution of 1-(4-(4-((3,4-diaminophenyl)thio)phenyl)piperazin-1-yl)ethan-1-one (Chemical Formula **3-12,** 0.090 g, 0.26 mmol) in 5% acetic acid in EtOH (2 mL) was added 1,3-bis(methoxycarbonyl)-S-methylisothiourea (134 mg, 0.65 mmol, 2.6 eq), and the mixture was warmed and stirred at 90°C for 24 hours. The reaction mixture was cooled sufficiently. MeOH (5 mL) was added.. The reaction mixture was filtered. Then the filtrate was washed with MeOH until the residual sulfurous odor of urea was completely removed and dried to afford methyl (5-((4-(4-acetylpiperazin-1-yl)phenyl)thio)-1H-benzo[d]imidazol-2-yl)carbamate (Chemical Formula **1-12,** 0.110 g) as a white solid (yield 92%).
¹H NMR (400 MHz, DMSO-d₆): δ 11.89(m 1H), 11.79(m, 1H), 7.33(m, 2H), 7.23(d, J=8.4Hz, 2H), 7.04(m, 1H), 6.95(d, J=8.8Hz, 2H), 3.74(s, 3H), 3.60(m, 7H), 3.17(m, 4H), 2.03(s, 3H)

### Preparation Example 13. Synthesis of Chemical Formula 1-13

The synthesis process of the thiobenzimidazole derivative of Chemical Formula 1-13 is shown by Reaction Scheme 13 presented below.

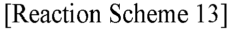

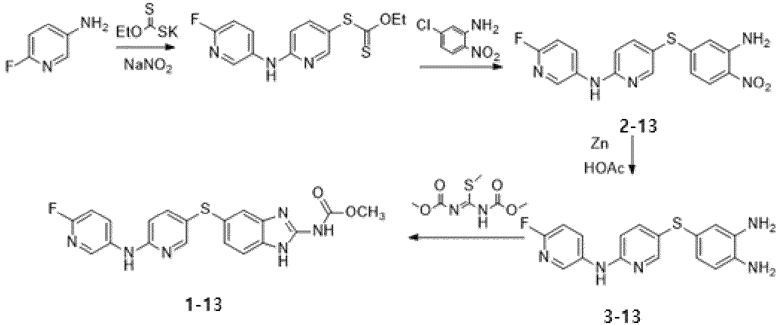

### 13.1. Synthesis of 5-((3-amino-4-nitrophenyl)thio)-N-(6-fluoropyridin-3-yl)pyridin-3-amine (Chemical Formula 2-13)

### (1) Synthesis of O-ethyl S-(5-((6-fluoropyridin-3-yl)amino)pyridin-3-yl) carbonodithioate

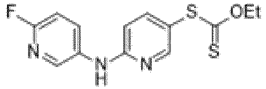

6-fluoropyridin-3-amine (0.297 g, 2.65 mmol) was dissolved in a solution of water (8 mL) and hydrochloric acid (0.4 mL) and then cooled in an ice bath. To the solution was slowly added dropwise a solution of NaNO₂ (186.3 mg, 2.7 mmol) in distilled water (1.5 mL) at the same temperature over 10 minutes. The temperature of the reaction mixture was adjusted to room temperature. To the reaction mixture was slowly added a previously prepared solution of potassium ethyl xanthate (528 mg, 3.3 mmol) in distilled water (2 ml) and the reaction mixture was stirred for 30 minutes. After the reaction was completed, the reaction mixture was extracted with ethyl acetate, the extract was washed with a small amount of water, and the solvent was removed. Then the crude product was separated by silica gel chromatography using ethyl acetate and hexane (1:3, v/v), and the solvent was removed to afford O-ethyl S-(5-((6-fluoropyridin-3-yl)amino)pyridin-3-yl) carbonodithioate (0.300 g) as a yellow solid (yield 36.2%).
¹H NMR (CDC1₃, 400 MHz) δ 8.26(s, 2H), 8.16(m, 1H), 7.64(d, J=6.4Hz, 1H), 6.99(m, 1H), 6.76(d, J=8.4Hz, 1H), 6.62(s, 1H), 4.66(q, 2H), 1.41(t, 3H)

### (2) Synthesis of 5-((3-amino-4-nitrophenyl)thio)-N-(6-fluoropyridin-3-yl)pyridin-3-amine (Chemical Formula 2-13)

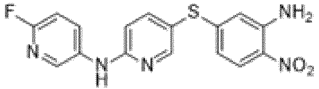

O-ethyl S-(5-((6-fluoropyridin-3-yl)amino)pyridin-3-yl) carbonodithioate (0.28 g, 0.91 mmol) was dissolved in a mixed solution of THF (10 ml), methanol (15 ml), and H₂O (3 ml). To the solution were added 5-chloro-2-nitroaniline (159 mg, 0.92 mmol) and NaOH (146 mg, 3.6 mmol). The reaction mixture was stirred at 80 to 90°C overnight. After the reaction was completed, the solvent was removed. The resulting product was extracted with ethyl acetate, then the extract was washed with water and dried to remove the solvent. The resulting reaction mixture was separated by silica gel column chromatography using ethyl acetate and hexane (3: 1, v/v) to afford 5-((3-amino-4-nitrophenyl)thio)-N-(6-fluoropyridin-3-yl)pyridin-3-amine (Chemical Formula **2-13,** 0.20 g) as a solid compound (yield 61.9%).
¹H NMR (CDC1₃, 400 MHz) δ 9.75(s, 1H), 8.52(s, 1H), 8.33(s, 2H), 7.89(d, J=9.2Hz, 1H), 7.78(d, J=8.4Hz, 1H), 7.45(s, 2H), 7.16(m, 1H), 6.97(d, J=8.8Hz, 1H), 6.51(s, 1H), 6.36(d, J=9.2Hz, 1H)

### 13.2. Synthesis of 4-((5-((6-fluoropyridin-3-yl)amino)pyridin-3-yl)thio)benzene-1,2-diamine (Chemical Formula 3-13)

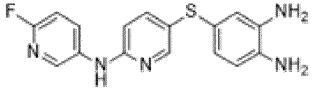

5-((3-amino-4-nitrophenyl)thio)-N-(6-fluoropyridin-3-yl)pyridin-3-amine (Chemical Formula **2-13,** 0.2 g, 0.56 mmol) was dissolved in CH₃COOH (3 ml). To the solution was slowly added zinc (160 mg, 2.46 mmol). The mixture was then stirred at room temperature for 2 hours. After the reaction was completed, the solvent was removed. The filtrate was extracted with ethyl acetate, then the extract was washed with water and dried to remove the solvent. The resulting reaction mixture was separated by silica gel column chromatography using ethyl acetate and hexane (9:1, v/v) to afford 4-((5-((6-fluoropyridin-3-yl)amino)pyridin-3-yl)thio)benzene-1,2-diamine (Chemical Formula **3-13,** 0.150 g) as a solid compound (yield 82%).
¹H NMR (DMSO, 400 MHz) δ 9.39(s, 1H), 8.45(s, 1H), 8.27(m, 1H), 8.07(s, 1H), 7.48(d, J=8.4Hz, 1H), 7.10(d, J=9.2Hz, 1H), 6.80(d, J=8.8Hz, 1H), 6.56(s, 1H), 6.48(m 2H), 4.69(s, 2H), 4.61(s, 2H)

### 13.3. Synthesis of methyl (5-((5-((6-fluoropyridin-3-yl)amino)pyridin-3-yl)thio)-1H-benzo[d]imidazol-2-yl)carbamate (Chemical Formula 1-13)

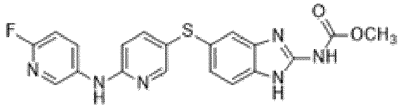

A mixture of 4-((5-((6-fluoropyridin-3-yl)amino)pyridin-3-yl)thio)benzene-1,2-diamine (Chemical Formula **3-13,** 0.140 g, 0.43 mmol), 1,3-bis(methoxycarbonyl)-S-methyl-isothiourea (240 mg, 1.15 mmol) and 5% acetic acid (3 ml) in ethanol was reacted overnight at a reaction temperature of 80 to 90°C to produce crystals. The mixture was cooled and filtered. The filtrate was washed with 50 ml of methanol and dried to afford methyl (5-((5-((6-fluoropyridin-3-yl)amino)pyridin-3-yl)thio)-1H-benzo[d]imidazol-2-yl)carbamate (Chemical Formula **1-13,** 0.150 g) as a solid compound (yield 85%).
¹H NMR (CDC1₃, 400 MHz) δ 11.85(m, 1H), 11.33(m, 1H), 9.51(s, 1H), 8.47(s, 1H), 8.28(m, 1H), 8.23(s, 1H), 7.63(d, J=9.2Hz, 1H), 7.33(m, 2H), 7.11(m, 2H), 6.85(d, J=9.2Hz, 1H), 3.74(s, 3H)

### Preparation Example 14. Synthesis of Chemical Formula 1-14

The synthesis process of the thiobenzimidazole derivative of Chemical Formula 1-14 is shown by Reaction Scheme 14 presented below.

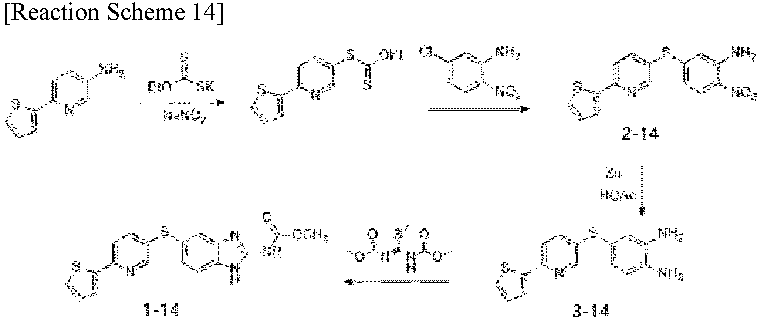

### 14.1. Synthesis of 2-nitro-5-((6-(thiophen-2-yl)pyridin-3-yl)thio)aniline (Chemical Formula 2-14)

### (1) Synthesis of O-ethyl S-(6-(thiophen-2-yl)pyridin-3-yl) carbonodithioate

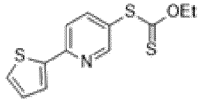

6-(thiophen-2-yl)pyridin-3-amine (0.9 g, 5.1 mmol) was dissolved in a mixed solution of water (14 mL) and C-HCl (0.75 mL). The reaction mixture was cooled in an ice bath. To the reaction mixture was slowly added sodium nitrate (0.4 g, 5.7 mmol) and water (3 mL) while the temperature was maintained. The reaction mixture was warmed to room temperature. A previously prepared solution of potassium ethyl xanthate (1.23 g, 5.97 mmol) in water (3 ml) was added, and the reaction mixture was stirred for 30 minutes. When a solid was formed, the temperature was raised to 50°C and a mixed solution of water and C-HCl (14 mL + 0.75 mL) was further added. After the reaction was completed, the mixture was extracted with ethyl acetate. The extract was washed with water, and then separated by silica gel chromatography using ethyl acetate and hexane (9:1, v/v) to give O-ethyl S-(6-(thiophen-2-yl)pyridin-3-yl) carbonodithioate (0.60 g) as a yellow solid (yield 42%).
¹H NMR (400 MHz, CDCl₃): δ 8.61(s, 1H), 7.82(d, J=8.8Hz, 1H), 7.74(d, J=8.0Hz, 1H), 7.68(s, 1H), 7.49(d, J=5.1Hz, 1H), 7.28(s, 1H), 7.16(m, 1H), 4.66(q, 2H), 1.38(t, 3H)

### (2) Synthesis of 2-nitro-5-((6-(thiophen-2-yl)pyridin-3-yl)thio)aniline (Chemical Formula 2-14)

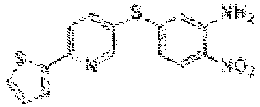

O-ethyl S-(6-(thiophen-2-yl)pyridin-3-yl) carbonodithioate (0.500 g, 1.78 mmol) was dissolved in a mixed solution of THF (18 ml), methanol (30 ml), and H₂O (6 ml). To the solution were added 5-chloro-2-nitroaniline (312 mg, 1.79 mmol) and NaOH (288 mg, 7.2 mmol) and the reaction mixture was stirred at 100°C for 8 hours. After the reaction was completed, the solvent was removed by evaporation under reduced pressure. The resulting product was extracted with ethyl acetate, then was washed with water, and dried over magnesium anhydride to remove the solvent. The resulting reaction mixture was separated by silica gel column chromatography using ethyl acetate and hexane (1:1, v/v) to afford 2-nitro-5-((6-(thiophen-2-yl)pyridin-3-yl)thio)aniline (Chemical Formula **2-14,** 0.050 g) as a solid compound (yield 25.6%).
¹H NMR (400 MHz, CDCl₃): δ 8.70(s, 1H), 8.04(d, J=9.2Hz, 1H), 7.85(m, 1H), 7.74(d, J=8.0Hz, 1H), 7.68(s, 1H), 7.51(s, 1H), 7.18(m, 1H), 6.50(d, J=8.8Hz, 1H), 6.41(s, 1H), 6.01(s, 2H)

### 14.2. Synthesis of 4-((6-(thiophen-2-yl)pyridin-3-yl)thio)benzene-1,2-diamine (Chemical Formula 3-14)

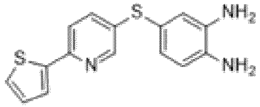

2-nitro-5-((6-(thiophen-2-yl)pyridin-3-yl)thio)aniline (Chemical Formula **2-14,** 120 mg, 0.36 mmol) was dissolved in CH₃COOH (2 ml). To the solution was slowly added in small portions zinc powder (100 mg) and then the mixture was stirred at room temperature for 2 hours. The mixture was then filtered and washed thoroughly with methylene chloride. The filtrate was collected, washed and neutralized with saturated sodium bicarbonate solution, and the solvent was removed. The mixture was again dissolved in ethyl acetate, then the solvent was removed and the residue was separated to obtain 4-((6-(thiophen-2-yl)pyridin-3-yl)thio)benzene-1,2-diamine (Chemical Formula **3-14,** 95 mg) as a solid compound (yield 87%).
¹H NMR (400 MHz, CDCl₃): δ 8.52(s, 1H), 7.83(s, 1H), 7.62(d, J=5.2Hz, 1H), 7.45(s, 2H), 7.33(m 1H), 6.78(d, J=3.2Hz, 1H), 6.31(s, 1H), 6.24(d, J=2.8Hz, 1H)

### 14.3. Synthesis of methyl (5-((6-(thiophen-2-yl)pyridin-3-yl)thio)-1H-benzo[d]imidazol-2-yl)carbamate (Chemical Formula 1-14)

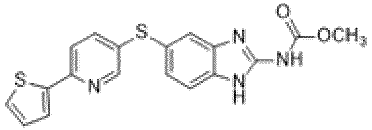

4-((6-(thiophen-2-yl)pyridin-3-yl)thio)benzene-1,2-diamine (Chemical Formula **3-14,** 0.060 g, 0.2 mmol) was added in 5% acetic acid in EtOH (2 mL) together with 1,3-bis(methoxycarbonyl)-S-methylisothiourea (120 mg, 0.58 mmol), and the mixture was warmed and stirred at 90°C for 24 hours. The reaction mixture was cooled sufficiently to give a solid. The solid was filtered and washed with MeOH. The filtrate was washed with MeOH until the residual sulfurous odor of urea was completely removed and dried to afford methyl (5-((6-(thiophen-2-yl)pyridin-3-yl)thio)-1H-benzo[d]imidazol-2-yl)carbamate (Chemical Formula **1-14,** 0.07 g) as a white solid (yield 78%).
¹H NMR (400 MHz, DMSO-d₆): δ 12.01(m,1H), 11.57(m, 1H), 8.34(s, 1H), 7.85(d, J=8.4Hz, 1H), 7.75(m, 1H), 7.63(d, J=4.8Hz, 1H), 7.47(s, 2H), 7.24(d, J=1.6Hz, 1H), 7.22(d, J=1.6Hz, 1H), 7.16(d, J=4.0Hz, 1H), 7.14(d, J=8.8Hz, 1H), 3.76(s, 3H)

### Preparation Example 15. Synthesis of Chemical Formula 1-15

The synthesis process of the thiobenzimidazole derivative of Chemical Formula 1-15 is shown by Reaction Scheme 15 presented below.

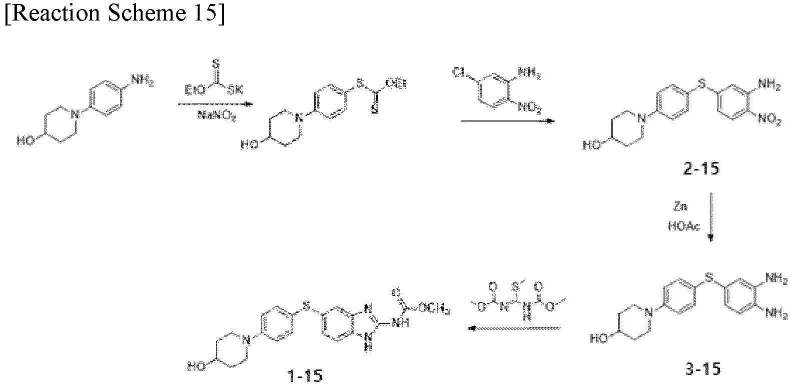

### 15.1. Synthesis of 1-(4-((3-amino-4-nitrophenyl)thio)phenyl)piperidin-4-ol (Chemical Formula 2-15)

### (1) Synthesis of O-ethyl S-(4-(4-hydroxypiperidin-1-yl)phenyl) carbonodithioate

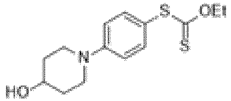

1-(4-Aminophenyl)piperidin-4-ol (0.9 g, 5.0 mmol) was dissolved in a mixed solution of water (3.5 mL) and C-HCl (0.72 mL), and the reaction mixture was cooled at 0°C in an ice bath. To the solution was slowly added a previously prepared solution of sodium nitrate (0.39 g, 5.6 mmol) and water (3 mL) while the temperature was maintained. The reaction mixture was warmed to room temperature. To the reaction mixture was slowly added a previously prepared solution of potassium ethyl xanthate (1.18 g, 5.73 mmol) in water (3 ml) and the reaction mixture was stirred for 20 minutes. When a solid was formed and thus stirring became difficult, the temperature was raised to 50°C or a mixed solution of water and C-HCl (14 mL + 0.75 mL) was further added. After the reaction was completed, the mixture was extracted with ethyl acetate. Then the resulting mixture was washed with water, and separated by silica gel chromatography using ethyl acetate and hexane (1: 1, v/v) to obtain O-ethyl S-(4-(4-hydroxypiperidin-1-yl)phenyl) carbonodithioate (1.1 g) as a light yellow solid (yield 78.6%). TLC with ethyl acetate and hexane (9:1, v/v) eluent showed good purity.
¹H NMR (CDCl₃, 400 MHz) δ 7.36(d, J=8.8Hz, 2H), 6.95(d, J=8.8Hz, 2H), 4.64(q, 2H), 3.94(m, 1H), 3.71(m, 2H), 3.07(m, 2H), 2.07(m, 2H), 1.71(m, 2H), 1. 32(t, 3H)

### (2) Synthesis of 1-(4-((3-amino-4-nitrophenyl)thio)phenyl)piperidin-4-ol (Chemical Formula 2-15)

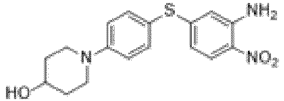

O-ethyl S-(4-(4-hydroxypiperidin-1-yl)phenyl) carbonodithioate (1.0 g, 3.37 mmol) was dissolved in a mixture of THF (33 ml), methanol (56 ml), H₂O (11.2 ml). To the solution were sequentially added 5-chloro-2-nitroaniline (0.5806 g, 3.38 mmol) and NaOH (536 mg, 13.4 mmol), and the mixture was reacted at 90°C overnight. After the reaction was completed, the mixture was cooled to room temperature, the solvent was removed by evaporation under reduced pressure to 2/3 and the recrystallized material was filtered and the resulting material was washed with a small amount of distilled water to obtain 1-(4-((3-amino-4-nitrophenyl)thio)phenyl)piperidin-4-ol (Chemical Formula 2-15, 0.98 g) as a yellow solid (yield 84%).
¹H NMR (CDCl₃, 400 MHz) δ 7.86(d, J=9.2Hz, 1H), 7.45(s, 2H), 7.36(d, J=8.8Hz, 2H), 6.51(d, J=8.8Hz, 2H), 6.451(s, 1H), 6.26(d, J=9.2Hz, 1H), 4.45(s, 1H), 3.67(m, 3H), 2.99(m, 2H), 1.84(m, 2H), 1.46(m, 2H)

### 15.2. Synthesis of 1-(4-((3,4-diaminophenyl)thio)phenyl)piperidin-4-ol (Chemical Formula 3-15)

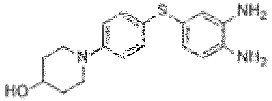

1-(4-((3-amino-4-nitrophenyl)thio)phenyl)piperidin-4-ol (Chemical Formula 2-15, 0.601 g, 1.74 mmol) was dissolved in CH₃COOH (5 ml). To the solution was slowly added in small portions zinc powder (200 mg, 2 eq) while care was taken not to cause an exothermic reaction, and then the mixture was stirred at room temperature for 2 hours. The reaction mixture was filtered to remove the zinc powder and the resulting mixture was washed with ethyl acetate. The filtrate was washed and neutralized with saturated aqueous sodium bicarbonate solution, and the solvent was removed by evaporation under reduced pressure, leaving a small amount of solvent to crystallize. The resulting solid was filtered and washed with a small amount of ethyl acetate and hexane (1:1, v/v) to afford 1-(4-((3,4-diaminophenyl)thio)phenyl)piperidin-4-ol (Chemical Formula 3-15, 0.45 g) as a solid compound (82% yield).
¹H NMR (DMSO, 400 MHz) δ 7.04(d, J=8.8Hz, 2H), 6.85(d, J=8.8Hz, 2H), 6.57(s, 1H), 6.46(s, 2H), 4.63(m, 3H), 4.55(s, 2H), 3.62(m, 1H), 3.48(m, 2H), 2.80(m, 2H), 1.79(m, 2H), 1.42(m, 2H)

### 15.3. Synthesis of methyl (5-((4-(4-hydroxypiperidin-1-yl)phenyl)thio)-1H-benzo[d]imidazol-2-yl)carbamate (Chemical Formula 1-15)

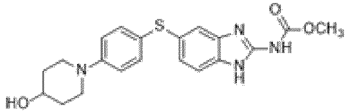

To a solution of 1-(4-((3,4-diaminophenyl)thio)phenyl)piperidin-4-ol (Chemical Formula 3-15, 0.10 g, 0.317 mmol) in 5% acetic acid in EtOH (4 mL) was added 1,3-bis(methoxycarbonyl)-S-methylisothiourea (200 mg, 0.97 mmol), and the mixture was heated and stirred at 90°C for 24 hours. The reaction mixture was cooled sufficiently and allowed to stand at room temperature to obtain a solid. The solid was filtered and simultaneously washed with MeOH. The residue was washed with MeOH until the sulfurous odor of the residual urea was completely removed and dried to afford methyl (5-((4-(4-hydroxypiperidin-1-yl)phenyl)thio)-1H-benzo[d]imidazol-2-yl)carbamate (Chemical Formula 1-15, 0.11 g) as a white solid (yield 87%).
¹H NMR (400 MHz, DMSO-d₆): δ 11.57(m, 2H), 7.32(m, 1H), 7.29(s, 1H), 7.21(d, J=8.8Hz, 2H), 7.03(m, 1H), 6.92(d, J=8.8Hz, 2H), 4.64(s, 1H), 3.74(s, 3H), 3.65(m, 1H), 3.55(m, 2H), 2.90(m, 2H), 1.82(m, 2H), 1.45(m, 2H)

### Preparation Example 16. Synthesis of Chemical Formula 1-16

The synthesis process of the thiobenzimidazole derivative of Chemical Formula 1-16 is shown by Reaction Scheme 16 presented below.

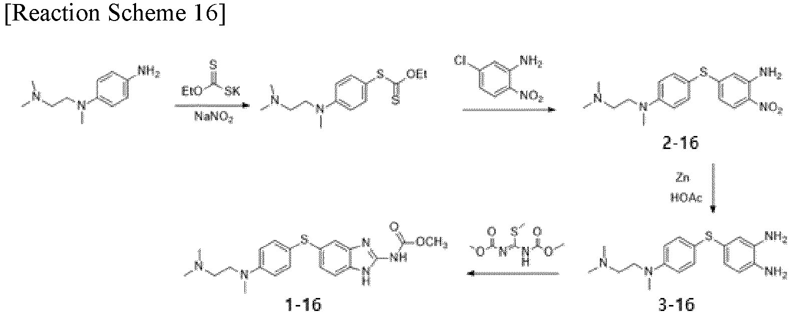

### 16.1. Synthesis of N1-(4-((3-amino-4-nitrophenyl)thio)phenyl)-N1,N2,N2-trimethylethane-1,2-diamine (Chemical Formula 2-16)

### (1) Synthesis of S-(4-((2-(dimethylamino)ethyl)(methyl)amino)phenyl) O-ethyl carbonodithioate

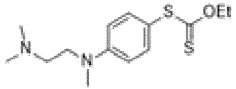

1-N-[2-(dimethylamino)ethyl]-1-N-methylbenzene-1,4-diamine (1.1 g, 5.7 mmol) was dissolved in a mixed solution of water (15 mL) and C-HCl (0.81 mL), and the reaction mixture was cooled at 0°C in an ice bath. To the reaction mixture was slowly added a previously prepared solution of sodium nitrate (0.44 g, 6.38 mmol) in water (4 mL) while the temperature was maintained, and the mixture was reacted for 20 minutes. The reaction mixture was warmed to room temperature. To the reaction mixture was slowly added a previously prepared a solution of potassium ethyl xanthate (1.33 g, 8.35 mmol) in water (5 ml) and the reaction mixture was stirred for 30 minutes. When a solid was formed and thus stirring became difficult, the temperature was raised to 50°C or a mixed solution of water and C-HCl (14 mL + 0.75 mL) was further added. After the reaction was completed, the mixture was extracted with ethyl acetate. Then the resulting mixture was washed with water, and separated by silica gel chromatography using the eluent of ethyl acetate and hexane (1:1, v/v) and ethyl acetate and hexane (9:1, v/v) to give S-(4-((2-(dimethylamino)ethyl)(methyl)amino)phenyl) O-ethyl carbonodithioate (0.86 g) as a light yellow solid (yield 51%).
¹H NMR (CDCl₃, 400 MHz) δ 7.32(d, J=8.4Hz, 2H), 6.71(d, J=8.8Hz, 2H), 4.64(q, 2H), 3.52(t, 2H), 3.02(s, 3H), 2.50(t, 2H), 2.31(s, 6H), 1.36(t, 3H)

### (2) Synthesis of N1-(4-((3-amino-4-nitrophenyl)thio)phenyl)-N1,N2,N2-trimethylethane-1,2-diamine (Chemical Formula 2-16)

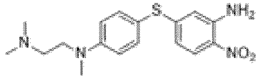

S-(4-((2-(dimethylamino)ethyl)(methyl)amino)phenyl) O-ethyl carbonodithioate (0.7 g, 2.35 mmol) was dissolved in a mixed solution of THF (23 ml), methanol (39 ml), and H₂O (7.8 ml). To the solution was sequentially added 5-chloro-2-nitroaniline (0.406 g, 2.35 mmol) and NaOH (0.375 g, 9.4 mmol), and the mixture was reacted at 90°C overnight. After the reaction was completed, the mixture was cooled to room temperature, and the solvent was removed by evaporation under reduced pressure. The resulting product was dissolved in ethyl acetate solvent and washed with water. The crude product was separated by silica gel column chromatography using ethyl acetate and hexane (9:1, v/v/) and ethyl acetate and hexane (5:1, v/v) eluent, and then purified to afford N1-(4-((3-amino-4-nitrophenyl)thio)phenyl)-N1,N2,N2-trimethylethane-1,2-diamine (Chemical Formula 2-16, 0.68 g) as a light yellow solid (yield 84%).
¹H NMR (400 MHz, CDCl₃): δ 7.858(d, J=9.2Hz, 1H), 7.449s, 2H), 7.34(d, J=8.4hz, 2H), 6.78(d, J=8.8Hz, 2H), 6.50(s, 1H), 6.25(d, J=8.8Hz, 1H), 3.46(m, 2H), 2.96(s, 3H), 2.39(m, 2H), 2.21(s, 6H)

### 16.2. Synthesis of 4-((4-((2-(dimethylamino)ethyl)(methyl)amino)phenyl)thio)benzene-1,2-diamine (Chemical Formula 3-16)

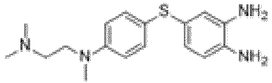

N1-(4-((3-amino-4-nitrophenyl)thio)phenyl)-N1,N2,N2-trimethylethane-1,2-diamine (Chemical Formula **2-16**, 0.3 g, 0.87 mmol) was dissolved in CH₃COOH (5 ml). To the solution was slowly added in small portions zinc powder (200 mg, 2 eq) while care was taken not to cause an exothermic reaction, and then the mixture was reacted at room temperature for 1 hour. The reaction mixture was filtered to remove the zinc powder and washed with ethyl acetate. The filtrate was washed continuously with water and neutralized with saturated aqueous sodium bicarbonate solution, and then the solvent was removed by evaporation under reduced pressure. The crude product was separated by silica gel column chromatography using ethyl acetate and methanol (5:1, v/v) followed by methylene chloride and methanol (9:1, v/v) eluent to afford 4-((4-((2-(dimethylamino)ethyl)(methyl)amino)phenyl)thio)benzene-1,2-diamine (Chemical Formula 3-16, 0.25 g) as a light yellow solid (yield 91%).
¹H NMR (CDCl₃, 400 MHz) δ 7.30(d, J=8.8Hz, 2H), 6.66(m, 5H), 3.48(m, 2H), 3.29(s, 4H), 2.97(s, 3H), 2.51(m, 2H), 2.31(s, 6H)

### 16.3 Synthesis of methyl (5-((4-((2-(dimethylamino)ethyl)(methyl)amino)phenyl)thio)-1H-benzo[d]imidazol-2-yl)carbamate (Chemical Formula 1-16)

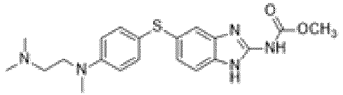

4-((4-((2-(dimethylamino)ethyl)(methyl)amino)phenyl)thio)benzene-1,2-diamine (Chemical Formula **3-16**, 0.06 g, 0.18 mmol) was added in 5% acetic acid in EtOH (2 mL) together with 1,3-bis(methoxycarbonyl)-S-methylisothiourea (0.12 g, 0.58 mmol), the mixture was warmed and stirred at 90°C for 16 hours, and then the solvent was removed by evaporation under reduced pressure. The reaction product was separated by silica gel column chromatography with an eluent of ethyl acetate and methanol (3: 1, v/v) to obtain methyl (5-((4-((2-(dimethylamino)ethyl)(methyl)amino)phenyl)thio)-1H-benzo[d]imidazol-2-yl)carbamate (Chemical Formula **1-16**, 0.045 g) as a light gray solid (yield 59%).
¹H NMR (400 MHz, DMSO-d₆): δ 10.57(m, 1H), 7.39(d, J=8.8Hz, 2H), 7.30(m, 2H), 7.16(d, J=8.4Hz,1H), 6.69(d, J=8.4Hz, 2H), 3.86(s, 3H), 3.48(m, 2H), 2.99(s, 3H), 2.49(m, 2H), 2.31(s, 7H)

### Preparation Example 17. Synthesis of Chemical Formula 1-17

The synthesis process of the thiobenzimidazole derivative of Chemical Formula 1-17 is shown by Reaction Scheme 16 presented below.

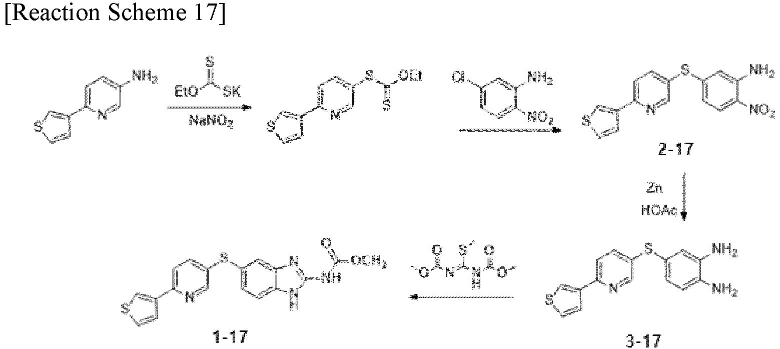

### 17.1. Synthesis of 2-nitro-5-((6-(thiophen-3-yl)pyridin-3-yl)thio)aniline (Chemical Formula 2-17)

### (1) Synthesis of O-ethyl S-(6-(thiophen-3-yl)pyridin-3-yl) carbonodithioate

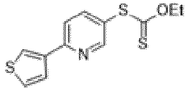

6-(thiophen-2-yl)pyridin-3-amine (0.9 g, 5.1 mmol) was dissolved in a mixed solution of water (13 mL) and C-HCl (0.75 mL), and the reaction mixture was cooled at 0°C in an ice bath. To the solution was slowly added dropwise a previously prepared solution of sodium nitrate (0.40 g, 5.7 mmol) in water (3 mL) while the temperature was maintained. The reaction mixture was warmed to room temperature. To the reaction mixture was added dropwise a previously prepared solution of potassium ethyl xanthate (1.23 g, 5.97 mmol) in water (5 ml) and the mixture was stirred for 30 minutes. When a solid was formed, and thus stirring became difficult, the temperature was raised to 50°C or a mixed solution of water and C-HCl (14 mL + 0.75 mL) was further added. After the reaction was completed, the mixture was extracted with ethyl acetate, then washed with water, and separated by silica gel chromatography using the eluent of ethyl acetate and hexane (1:1, v/v) followed by ethyl acetate and hexane (9:1, v/v) to obtain O-ethyl S-(6-(thiophen-3-yl)pyridin-3-yl) carbonodithioate (0.61 g) as a yellow solid (yield 42%).
¹H NMR (CDC1₃, 400 MHz) δ 8.67(s, 1H), 8.02(s, 1H), 7.84(d, J=6.0Hz, 1H), 7.72(m, 2H), 7.45(m, 1H), 4.67(q, 2H), 1.38(t, 3H)

### (2) Synthesis of 2-nitro-5-((6-(thiophen-3-yl)pyridin-3-yl)thio)aniline (Chemical Formula 2-17)

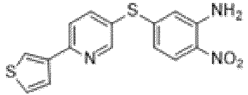

O-ethyl S-(6-(thiophen-3-yl)pyridin-3-yl) carbonodithioate (0.5 g, 1.78 mmol) was dissolved in a mixed solution of THF (18 ml), methanol (30 ml), and H₂O (6.0 ml). To the solution were sequentially added 5-chloro-2-nitroaniline (0.312 g, 1.79 mmol) and NaOH (0.288 g, 7.2 mmol), and the mixture was reacted at 90°C overnight. After the reaction was completed, the mixture was cooled to room temperature, and the solvent was removed by evaporation under reduced pressure. The resulting product was dissolved in ethyl acetate solvent and washed with water. The crude product was separated by silica gel column chromatography using ethyl acetate and hexane (9:1, v/v/) and ethyl acetate and hexane (5:1, v/v) eluent to afford 2-nitro-5-((6-(thiophen-3-yl)pyridin-3-yl)thio)aniline (Chemical Formula 2-17, 0.68 g) as a light yellow solid (yield 43%).

¹H NMR (400 MHz, CDCl₃): δ 8.74(s, 1H), 8.0(d. J=8.8Hz, 2H), 7.88(d, J=6.4Hz, 1H), 7.72(d, J=6.8Hz, 2H), 7.48(s, 1H), 6.49(d, J=8.8Hz, 1H), 6.40(s, 1H), 6.09(s, 2H),

### 17.2. Synthesis of 4-((6-(thiophen-3-yl)pyridin-3-yl)thio)benzene-1,2-diamine (Chemical Formula 3-17)

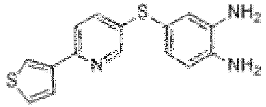

2-nitro-5-((6-(thiophen-3-yl)pyridin-3-yl)thio)aniline(Chemical Formula **2-17,** 0.22g, 0.67 mmol) was dissolved in CH₃COOH (3 ml). To the solution was slowly added in small portions zinc powder (0.15 g) while care was taken not to cause an exothermic reaction, and then the mixture was reacted at room temperature for 1 hour. The reaction mixture was filtered to remove the zinc powder and washed with ethyl acetate. The filtrate was washed and neutralized with saturated aqueous sodium bicarbonate solution, continuously washed with water, dried over anhydrous magnesium sulfate, and filtered. Then the solvent was removed by evaporation under reduced pressure. The crude product was separated by silica gel column chromatography using ethyl acetate and methanol (5:1, v/v) followed by methylene chloride and methanol (9:1, v/v) eluent to afford 4-((6-(thiophen-3-yl)pyridin-3-yl)thio)benzene-1,2-diamine (Chemical Formula **3-17,** 0.17 g) as a light yellow solid (yield 85%).
¹H NMR (400 MHz, CDCl₃): δ 8.50(s, 1H), 7.84(s, 1H), 7.62(d, J=5.2Hz, 1H), 7.48(s, 2H), 7.39(m 1H), 6.93(d, J=3.2Hz, 1H), 6.34(s, 1H), 6.27(d, J=2.8Hz, 1H)

### 17.3. Synthesis of methyl (5-((6-(thiophen-3-yl)pyridin-3-yl)thio)-1H-benzo[d]imidazol-2-yl)carbamate (Chemical Formula 1-17)

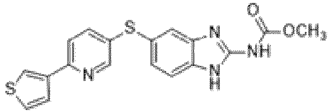

To a solution of 4-((6-(thiophen-3-yl)pyridin-3-yl)thio)benzene-1,2-diamine (Chemical Formula **3-17,** 0.06 g, 0.2 mmol) in 5% acetic acid in EtOH (2 mL) was added 1,3-bis(methoxycarbonyl)-S-methylisothiourea (0.103 g, 0.50 mmol), the mixture was warmed and stirred at 90°C for 20 hours and the solvent was removed by evaporation under reduced pressure. The reaction mixture was cooled sufficiently and allowed to stand at room temperature to obtain a solid. The solid was filtered and simultaneously washed with MeOH. The residue was washed with MeOH until the sulfurous odor of the residual urea was completely removed and dried to afford methyl (5-((6-(thiophen-3-yl)pyridin-3-yl)thio)-1H-benzo[d]imidazol-2-yl)carbamate (Chemical Formula **1-17,** 0.065 g) as a white solid (yield 78%).
¹H NMR (400 MHz, DMSO-d₆): δ 11.87(m, 1H), 11.82(m, 1H), 8.41(s, 1H), 8.14(s 1H), 7.81(d, J=8.4Hz, 1H), 7.09(s, 1H), 7.64(s, 1H), 7.63(d, J=8.0Hz, 1H), 7.57(s, 1H), 7.47(d, J=8.0Hz, 1H), 7.24(d, J=8.4Hz, 1H), 3.76(s. 3H)

### Preparation Example 18. Synthesis of Chemical Formula 1-18

The synthesis process of the thiobenzimidazole derivative of Chemical Formula 1-18 is shown by Reaction Scheme 18 presented below.

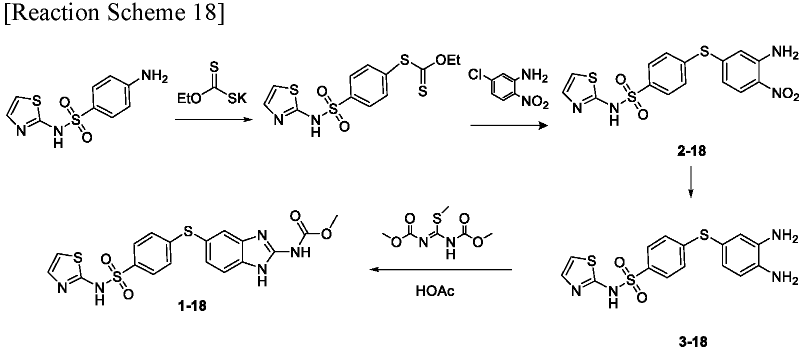

### 18.1. Synthesis of 4-((3,4-diaminophenyl)thio)-N-(thiazol-2-yl)benzenesulfonamide (Chemical Formula 3-18)

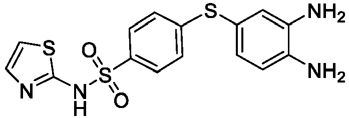

The above shown compound was prepared under the same conditions as Preparation Examples 2.1 and 2.2, except sulfathiazole was used instead of 4-[4-(1-methylethyl)-1-piperazinyl]benzenamine as the starting reactant.
The NMR data for 4-((3-amino-4-nitrophenyl)thio)-N-(thiazol-2-yl)benzenesulfonamide (Chemical Formula **2-18**) was as follows: ¹H NMR (400 MHz, DMSO-d₆): δ 12.64(s, 1H), 7.99(d, J=8.8Hz, 1H), 7.92(d, J=8.4Hz, 2H), 7.51(d, J=8.4Hz, 2H), 6.87(s, 1H), 6.58(s, 1H), 6.41(m, 4H)
The NMR data for 4-((3,4-diaminophenyl)thio)-N-(thiazol-2-yl)benzenesulfonamide (Chemical Formula **3-18**) was as follows: ¹H NMR (400 MHz, DMSO-d₆): δ 12.68(s, 1H), 7.72(d, J=8.8Hz, 2H), 7.11(m, 3H), 6.92(m, 2H), 6.76(d, J=8.0Hz, 1H), 6.50(s, 1H)

### 18.2. Synthesis of methyl (5-((4-(N-(thiazol-2-yl)sulfamoyl)phenyl)thio)-1H-benzo[d]imidazol-2-yl)carbamate (Chemical Formula 1-18)

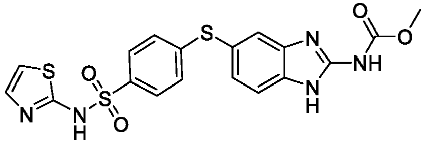

To a solution of 4-((3,4-diaminophenyl)thio)-N-(thiazol-2-yl)benzenesulfonamide (Chemical Formula **3-18,** 0.12 g, 0.32 mmol) in 5% acetic acid in EtOH (4 mL) was added 1,3-bis(methoxycarbonyl)-S-methylisothiourea (0.210 g, 1.01 mmol), and the mixture was warmed and stirred at 90°C for 4 hours. The reaction mixture was cooled sufficiently and allowed to stand at room temperature to obtain a solid. The solid was filtered and simultaneously washed with MeOH. The residue was washed with MeOH until the sulfurous odor of the residual urea was completely removed and dried to afford methyl (5-((4-(N-(thiazol-2-yl)sulfamoyl)phenyl)thio)-1H-benzo[d]imidazol-2-yl)carbamate (Chemical Formula **1-18,** 0.110 g) as a white solid (yield 75%).
¹H NMR (400 MHz, DMSO-d₆): δ 12.70(m 1H), 12.05(m, 1H), 11.56(m, 1H), 7.65(d, J=8.4Hz, 2H), 7.59(s, 1H), 7.51(d, J=8.0Hz, 1H), 7.25(m, 2H), 7.12(d, J=8.0Hz, 2H), 6.79(d, J=4.4Hz, 1H), 3.77(s, 3H)

### Preparation Example 19. Synthesis of Chemical Formula 1-19

The synthesis process of the thiobenzimidazole derivative of Chemical Formula 1-19 is shown by Reaction Scheme 19 presented below.

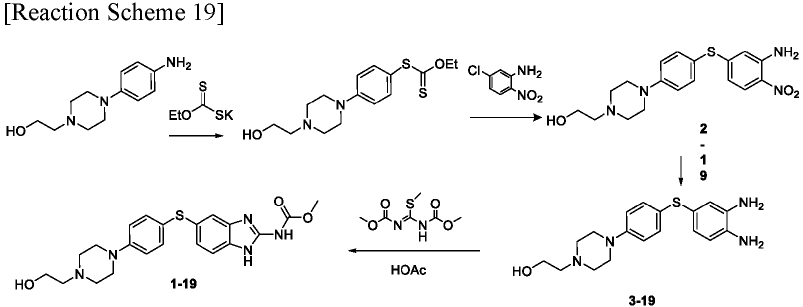

### 19.1. Synthesis of 2-(4-(4-((3,4-diaminophenyl)thio)phenyl)piperazin-1-yl)ethan-1-ol (Chemical Formula 3-19)

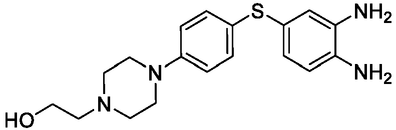

The above shown compound was prepared under the same conditions as Preparation Examples 2.1 and 2.2, except that 2-(4-(4-Aminophenyl)piperazin-1-yl)ethanol was used instead of 4-[4-(1-Methylethyl)-1-piperazinyl]benzenamine as the starting reactant.
The NMR data for 2-(4-(4-((3-amino-4-nitrophenyl)thio)phenyl)piperazin-1-yl)ethan-1-ol (Chemical Formula **2-19**) was as follows: ¹H NMR (400 MHz, DMSO-d₆): δ 7.98(m, 1H), 7.45(d, J=9.6Hz, 2H), 6.99(d, J=8.8Hz, 2H), 6.44(m, 1H), 6.29(m, 1H), 6.02(s, 2H), 3.73(m, 2H), 3.35(m, 4H), 2.73(m, 4H), 2.86(m, 2H)
The NMR data for 2-(4-(4-((3,4-diaminophenyl)thio)phenyl)piperazin-1-yl)ethan-1-ol (Chemical Formula **3-19**) was as follows: ¹H NMR (400 MHz, DMSO-d₆): δ 7.04(d, J=8.4Hz, 2H), 6.85(d, J=8.4Hz, 2H), 6.569s, 1H), 6.46(s, 2H), 4.67(s, 2H), 4.58(s, 2H), 4.43(s, 1H), 3.51(s, 2H), 3.07(s, 4H), 2.51(m, 4H), 2.41(m, 2H)

### 19.2. Synthesis of methyl (5-((4-(N-(thiazol-2-yl)sulfamoyl)phenyl)thio)-1H-benzo[d]imidazol-2-yl)carbamate (Chemical Formula 1-19)

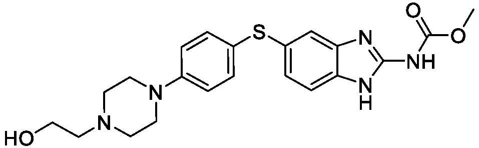

To a solution of 2-(4-(4-((3,4-diaminophenyl)thio)phenyl)piperazin-1-yl)ethan-1-ol (Chemical Formula **3-19,** 0.09 g, 0.260 mmol) in 5% acetic acid in EtOH (3 mL) was added 1,3-bis(methoxycarbonyl)-S-methylisothiourea (0.160 g, 0.78 mmol), and the mixture was warmed and stirred at 90°C for 5 hours. The reaction mixture was cooled sufficiently and allowed to stand at room temperature to obtain a solid. The solid was filtered and simultaneously washed with MeOH. The residue was washed with MeOH until the sulfurous odor of the residual urea was completely removed and dried to afford methyl 2-(5-((4-(4-(2-hydroxyethyl)piperazin-1-yl)phenyl)thio)-1H-benzo[d]imidazol-2-yl)acetate (Chemical Formula **1-19,** 0.08 g) as a white solid (yield 72%).
¹H NMR (400 MHz, DMSO-d₆): δ 11.52(m, 2H), 7.34(d, J=8.0Hz, 1H), 7.29(s, 1H), 7.22(d, J=8.8Hz, 2H), 7.03(d, J=8.4Hz, 1H), 6.93(d, J=8.8Hz, 2H), 4.50(m, 1H), 3.74(s, 3H), 3.55(m, 2H), 3.15(m, 4H), 2.55(m, 6H)

### Preparation Example 20. Synthesis of Chemical Formula 1-20

The synthesis process of the thiobenzimidazole derivative of Chemical Formula 1-20 is shown by Reaction Scheme 20 presented below.

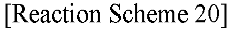

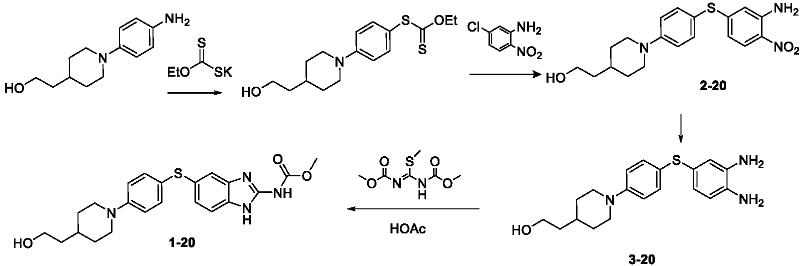

### 20.1. Synthesis of 2-(1-(4-((3,4-diaminophenyl)thio)phenyl)piperidin-4-yl)ethan-1-ol (Chemical Formula 3-20)

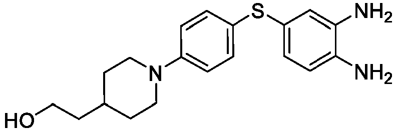

The above shown compound was prepared under the same conditions as Preparation Examples 2.1 and 2.2, except 2-(1-(4-Aminophenyl)piperidin-4-yl)ethan-1-ol was used instead of 4-[4-(1-Methylethyl)-1-piperazinyl]benzenamine as the starting reactant.
The NMR data for 2-(1-(4-((3-amino-4-nitrophenyl)thio)phenyl)piperidin-4-yl)ethan-1-ol (Chemical Formula **2-20**) was as follows: ¹H NMR (400 MHz, DMSO-d₆): δ 7.864(d, J=9.2Hz, 1H), 7.45(s, 2H), 7.36(d, J=8.8Hz, 2H), 7.04(d, J=8.8Hz, 2H), 6.52(s, 1H), 6.26(d, J=9.2Hz, 1H), 4.41(m, 1H), 3.83(d, J=12.8Hz, 2H), 3.48(m, 2H), 2.76(t, 2H), 1.76(d, J=12.8Hz, 2H), 1.60(m, 1H), 1.40(q, 2H), 1.22(m, 2H)
The NMR data for 2-(1-(4-((3,4-diaminophenyl)thio)phenyl)piperidin-4-yl)ethan-1-ol (Chemical Formula **3-20**) was as follows: ¹H NMR (400 MHz, DMSO-d₆): δ 7.24(d, J=8.8Hz, 2H), 6.87(d, J=8.8Hz, 2H), 6.71(m, 2H), 6.65(d, J=8.0Hz, 1H), 3.76(s, 2H), 3.69(d, J=12.4Hz, 2H), 3.38(bs, 4H), 2.71(m, 2H), 1.84(d, J=12.8Hz, 2H), 1.40(m, 3H), 1.27(m, 2H)

### 20.2. Synthesis of methyl (5-((4-(4-(2-hydroxyethyl)piperidin-1-yl)phenyl)thio)-1H-benzo[d]imidazol-2-yl)carbamate (Chemical Formula 1-20)

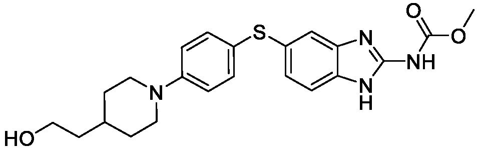

To a solution of 2-(1-(4-((3,4-diaminophenyl)thio)phenyl)piperidin-4-yl)ethan-1-ol (Chemical Formula **3-20,** 0.165 g, 0.45 mmol) in 5% acetic acid in EtOH (4 mL) was added 1,3-bis(methoxycarbonyl)-S-methylisothiourea (0.278 g, 1.35 mmol), and the mixture was warmed and stirrerd at 90°C for 3 hours. The reaction mixture was cooled sufficiently and allowed to stand at room temperature to obtain a solid. The solid was filtered and simultaneously washed with MeOH. The residue was washed with MeOH until the sulfurous odor of the residual urea was completely removed and dried to afford methyl (5-((4-(4-(2-hydroxyethyl)piperidin-1-yl)phenyl)thio)-1H-benzo[d]imidazol-2-yl)carbamate (Chemical Formula **1-20,** 0.152 g) as a white solid (yield 75%).
¹H NMR (400 MHz, DMSO-d₆): δ 11.71(m, 2H), 7.34(d, J=8.4Hz, 1H), 7.27(s, 1H), 7.21( d, J=8.4Hz, 2H), 7.02(d, J=8.4Hz, 1H), 6.92(d, J=8.4Hz, 2H), 4.39(m, 1H), 3.73(s, 3H), 3.70(m, 2H), 3.46(m, 2H), 2.66(m, 2H), 1.73(d, J=11.6Hz, 2H), 1.53(m, 1H), 1.38(m, 2H), 1.18(m, 2H)

### Example 1. Confirmation of Cell Viability for Thiobenzimidazole Derivatives against Breast Cancer Cell Lines

Human triple-negative breast cancer (TNBC) cell line MDA-MB-231 (Cell seeding numbers: 1(M231)×10⁴ cells/wells (confluency ≥ 25%) and HER2 positive breast cancer (HER2⁺ BC) cell line JIMT-1 (Cell seeding numbers: 1.2(JIMT)×10⁴ cells/wells (confluency ≥ 25%)) were used in the experiments.

The above mentioned cell lines were cultured under a 5% CO₂ environment at 37°C in Dulbecco's modified Eagle's medium (DMEM) containing 10% fetal bovine serum (FBS), streptomycin-penicillin (100 U/mL) and Fungizone (0.625 µg/mL), respectively.

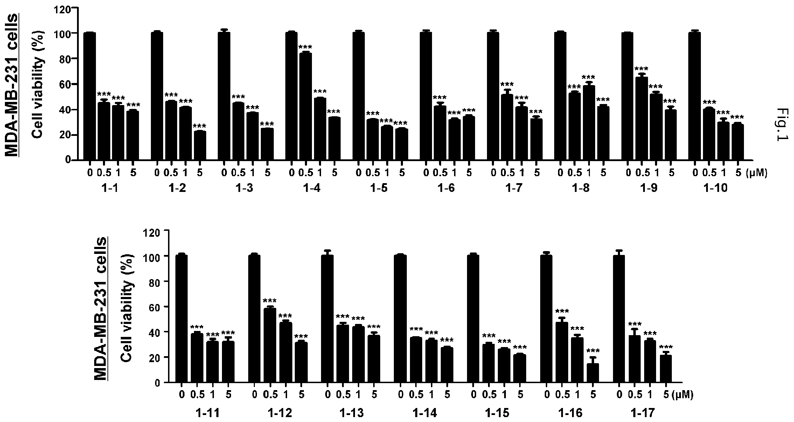

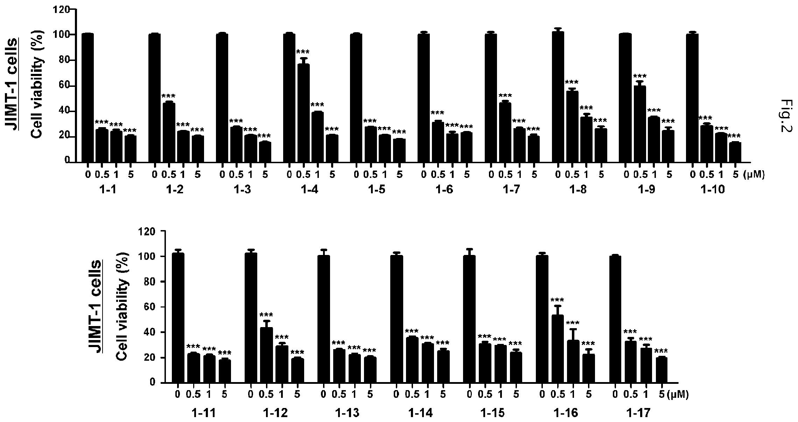

The human breast cancer cell lines MDA-MB-231 and JIMT-1 were treated with thiobenzimidazole derivatives of Chemical Formula 1-1 to Chemical Formula 1-17 at concentrations of 0, 0.5, 1 and 5 µM for 72 hours, respectively, and then cell viability was measured by MTS assay. The MTS assay was performed by attaching cells to 96-well plates for 24 hours, treating them with the above mentioned thiobenzimidazole derivatives for 72 hours, and then developing them with MTS (3-(4,5-dimethylthiazol-2-yl)-5-(3-carboxymethoxyphenyl)-2-(4-sulfophenyl)-2H-tetrazolium) for 4 hours, and measuring the absorbance at 490 nm using a Spectramax Plus384 microplate analyzer. The results for MDA-MB-231 are shown in Fig. 1 and the results for JIMT-1 are shown in Fig. 2.

As shown in the drawings, the results showed that the thiobenzimidazole derivatives of Chemical Formula 1-1 to 1-17 inhibited cell viability in a concentration-dependent manner in the human breast cancer cell lines MDA-MB-231 and JIMT-1.

### Example 2. Confirmation of Cell Viability and IC₅₀ Values for Thiobenzimidazole Derivatives against Breast Cancer Cell Lines

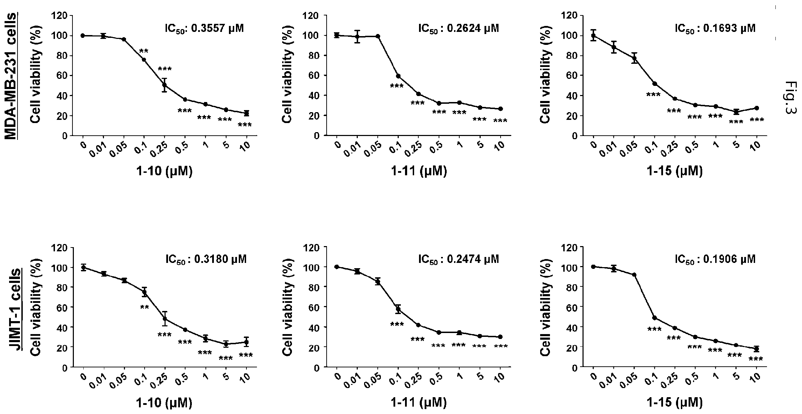

MDA-MB-231 and JIMT-1 cell lines were treated with the thiobenzimidazole derivatives of the present disclosure (Chemical Formula 1-10, 1-11 and 1-15) at concentrations of 0. 0.01, 0.05, 0.1, 0.1, 0.25, 0.5, 1, 5 and 10 µM, respectively, and then the cell viability and corresponding IC₅₀ values were determined. The preparation and experimentation methods for the cell lines were the same as those used in the Example 1 experiments. The results are shown in Fig. 3. It was found that the thiobenzimidazole derivatives of Formula 1-10, 1-11, and 1-15 had IC₅₀ values as low as 0.15 to 0.35 µM.

While the embodiments are described, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A thiobenzimidazole derivative or a pharmaceutically acceptable salt thereof represented by the following [Chemical Formula 1]: 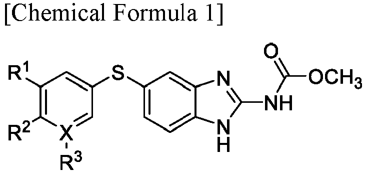 in Chemical Formula 1,
X is any one selected from C or N,
R¹, R² and R³ are each independently any one selected from the group consisting of hydrogen, halogen, -OR⁴, -NR⁵R⁶*,* a substituted or unsubstituted C₃ to C₂₀ cycloalkyl group, or a substituted or unsubstituted C₃ to C₂₀ heterocycloalkyl group, a substituted or unsubstituted C₃ to C₂₀ aryl group, a substituted or unsubstituted C₃ to C₂₀ heteroaryl group, and a substituted or unsubstituted sulfonamide group,
R⁴ is an alkyl group of C₁-C₁₂,
R⁵ and R⁶ are each independently any one selected from the group consisting of hydrogen, a C₁-C₁₂ alkyl group, -(CH₂)ₙ-N(R⁴)₂, a substituted or unsubstituted C₃ to C₂₀ aryl group, and a substituted or unsubstituted C₃ to C₂₀ heteroaryl group,
n is an integer from "0" to "6."

2. The thiobenzimidazole derivative or the pharmaceutically acceptable salt thereof of claim 1, wherein, in [Chemical Formula 1],
a substituted cycloalkyl group, heterocycloalkyl group, aryl group, heteroaryl group or sulfonamide group is substituted with one or more selected from the group consisting of a substituted or unsubstituted C₁-C₆ alkyl group, a substituted or unsubstituted sulfonyl group, a substituted or unsubstituted carbonyl group, a substituted or unsubstituted hydroxy group, and a heteroaryl group.

3. The thiobenzimidazole derivative or the pharmaceutically acceptable salt thereof of claim 1, wherein the thiobenzimidazole derivative represented by [Chemical Formula 1] is any one selected from the group consisting of the following compounds: 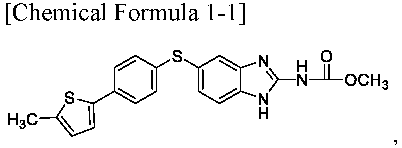 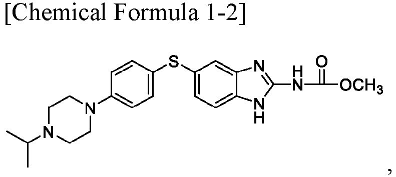 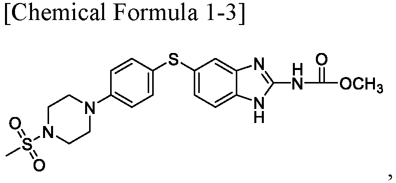 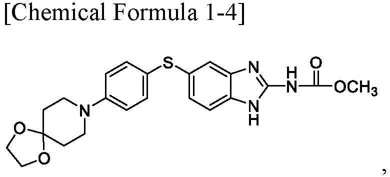 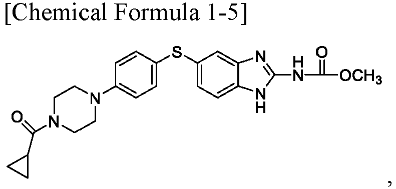 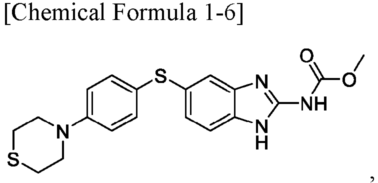 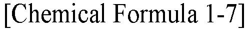 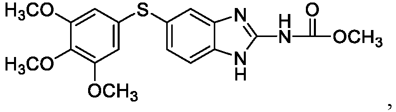 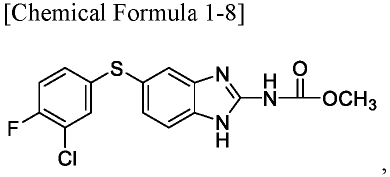 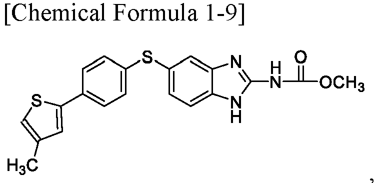 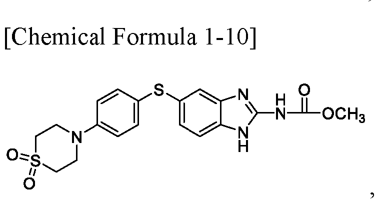 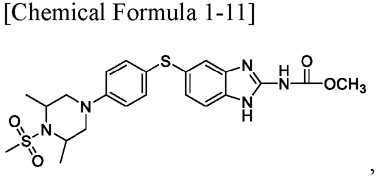 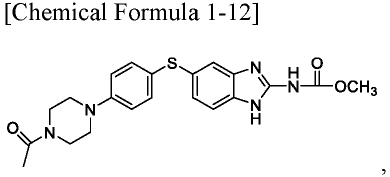 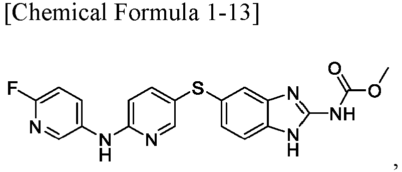 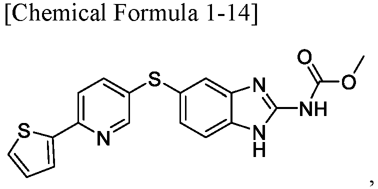 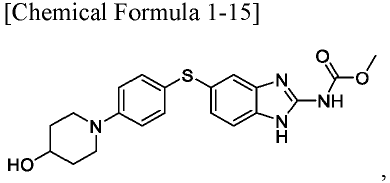 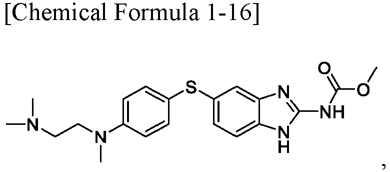 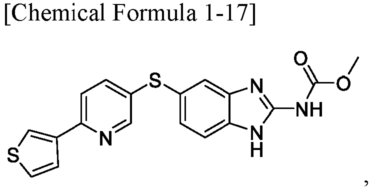 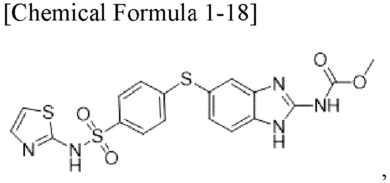 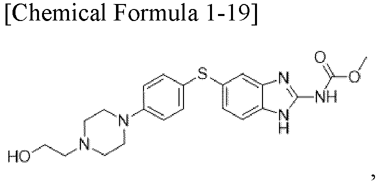 and

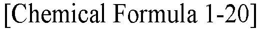

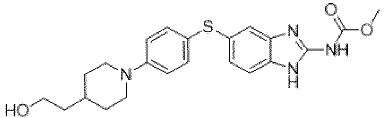

4. The thiobenzimidazole derivative or the pharmaceutically acceptable salt thereof of claim 1, wherein the thiobenzimidazole derivative represented by [Chemical Formula 1] is any one selected from the group consisting of the following compounds: 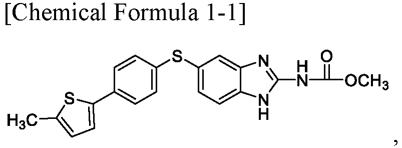 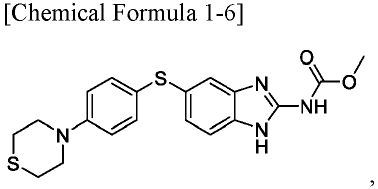 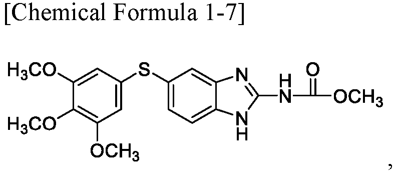 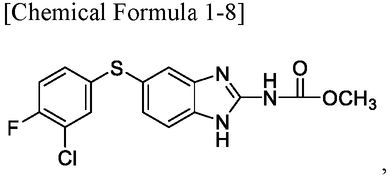 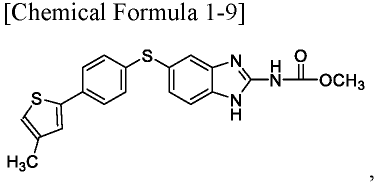 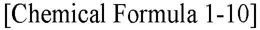 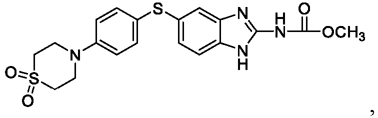 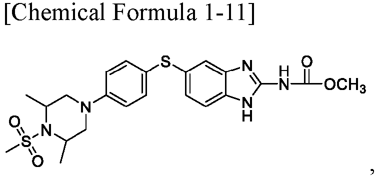 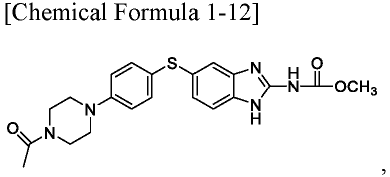 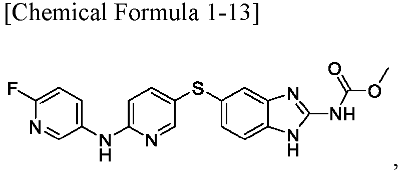 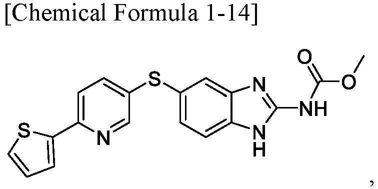 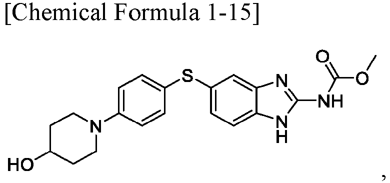 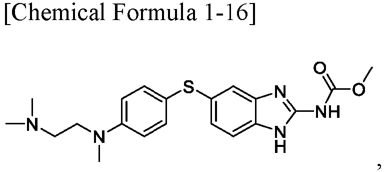 and

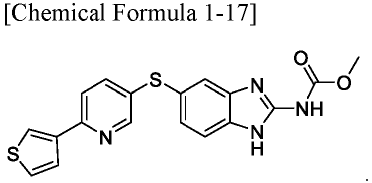

5. The thiobenzimidazole derivative or the pharmaceutically acceptable salt thereof of claim 1, wherein the thiobenzimidazole derivative inhibits tubulin polymerization.

6. The thiobenzimidazole derivative or the pharmaceutically acceptable salt thereof of claim 1, wherein the pharmaceutically acceptable salt of the thiobenzimidazole derivative is one or more selected from the group consisting of hydrochloride, bromate, sulfate, phosphate, nitrate, citrate, acetate, lactate, tartrate, maleate, gluconate, succinate, formate, trifluoroacetate, oxalate, fumarate, glutarate, adipate, methane sulfonate, benzenesulfonate, paratoluenesulfonate, camphorsulfonate, sodium salt, potassium salt, lithium salt, calcium salt, and magnesium salt.

7. The thiobenzimidazole derivative or the pharmaceutically acceptable salt thereof of claim 1, wherein the pharmaceutically acceptable salt of the thiobenzimidazole derivative is hydrochloride salt.

8. A pharmaceutical composition for preventing or treating a cancer, comprising the thiobenzimidazole derivative or the pharmaceutically acceptable salt thereof according to any one of claims 1 to 7 as an active ingredient.

9. The pharmaceutical composition of claim 8, wherein the pharmaceutical composition induces apoptosis by arresting a cell cycle of a cancer cell.

10. The pharmaceutical composition of claim 8, wherein the cancer is one or more selected from the group consisting of skin cancer, breast cancer, uterine cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, and bronchial cancer.

11. The pharmaceutical composition of claim 8, wherein the cancer is HER-2 positive breast cancer or triple-negative breast cancer.
